# EUROPEAN PATENT SPECIFICATION

(11) **EP 3 209 213 B1**
(45) Date of publication and mention of the grant of the patent: **10.05.2023**
(21) Application number: 15854615.0
(22) Date of filing: 20.10.2015
(51) Int. Cl.: A61B 6/02, A61B 6/00, A61B 6/12, A61B 90/00

(54) **SURGICAL DEVICES**
CHIRURGISCHE INSTRUMENTE
DISPOSITIFS MÉDICAUX

(30) Priority: 20.10.2014 US 201462065828 P
(43) Date of publication of application: 30.08.2017
(73) Proprietor: Body Vision Medical Ltd., 4720809 Ramat Hasharon (IL)
(72) Inventor: AVERBUCH, Dorian, Ramat Hasharon 4732157 (IL)
(74) Representative: Meissner Bolte Partnerschaft mbB
(86) International application number: PCT/IB2015/002148
(87) International publication number: WO 2016/067092

(56) References cited:
- US-A1- 2005 281 385
- US-A1- 2006 016 006
- US-A1- 2007 038 058
- US-A1- 2008 114 238
- US-A1- 2008 262 342
- US-A1- 2009 299 174
- US-A1- 2012 289 825

## Description

### FIELD OF THE INVENTION

The present invention relates to a system for generating an augmented fluoroscopy image. The present disclosure also relates to surgical devices and methods of use thereof.

### BACKGROUND OF INVENTION

Use of video-assisted thoracic surgery (VATS) during endoscopic surgery, as well as other fields of surgery, can be used during the treatment of various respiratory diseases.

Conventional systems configured to be used for such applications are known, for example, from US 2012/289825 A1 and US 2008/262342 A1.

### BRIEF SUMMARY OF INVENTION

The invention is defined by the appended claims.

### BRIEF DESCRIPTION OF THE DRAWINGS

The present invention will be further explained with reference to the attached figures. The figures constitute a part of this specification and include illustrative embodiments or aspects useful for understanding the present invention and illustrate various objects and features thereof. Specific functional details disclosed herein are not to be interpreted as limiting, but merely as a representative basis for teaching one skilled in the art to variously employ the present invention.
Figure 1 shows a surgical and diagnostic procedure flow chart according to the present disclosure.
Figure 2 is an illustration of an aspect of the method carried out by a system according to an embodiment of the present invention (e.g., showing an augmented fluoroscopy system and data flow).
Figures 3A and 3B are images illustrating an aspect of the method carried out by the system according to an embodiment of the present invention.
Figure 4 is a flow chart showing an aspect of the method carried out by the system according to an embodiment of the present invention (e.g., an anatomical structure enhancement flow chart).

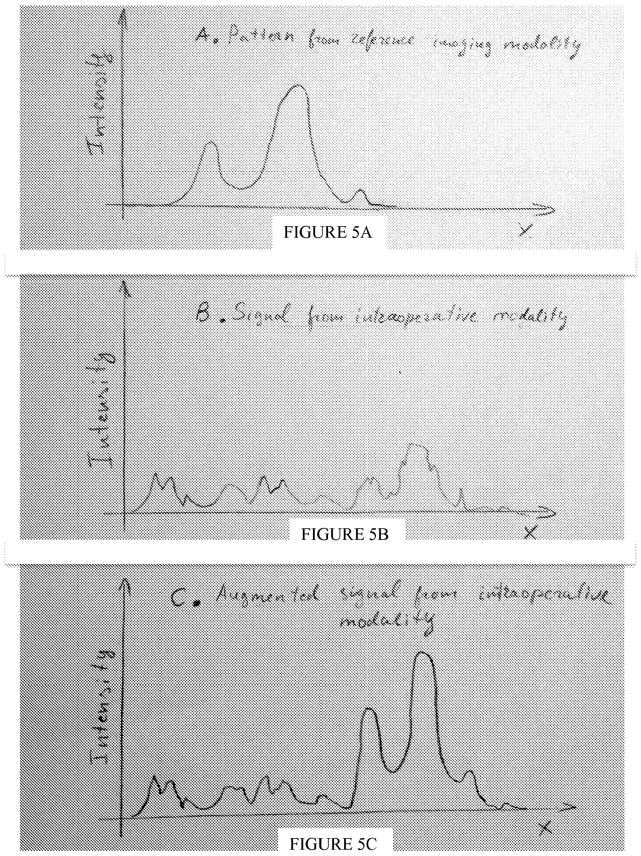

Figure 5 is an illustration showing an aspect of the method carried out by the system according to an embodiment of the present invention, illustrating three intensity measurements: (A) shows a pattern obtained from a reference imaging modality; (B) shows a signal from an intraoperative modality; and (C) shows an augmented signal from intraoperative modality. This illustration shows an aspect of the method carried out by the system according to an embodiment of the present invention, where the intensity measurements can be used for fine registration (i.e., template matching), based on at least one signal enhancement.
Figures 6A and 6B is a schematic drawing showing an aspect of the method carried out by the system according to an embodiment of the present invention, illustrating a fluoroscopic image.
Figure 7 is an aspect of the method carried out by the system according to an embodiment of the present invention, illustrating a registration step using (1) information pertaining to a bronchial airway tree, where the information is extracted from a preoperative image (e.g., a 2-dimensional or a 3-dimensional image; e.g., a CT scan) and (2) information pertaining to at least one airway, where the information is extracted from a fluoroscopic image(s) by use of an augmented bronchogram.
Figure 8 shows an aspect of the method carried out by the system according to an embodiment of the present invention, illustrating a fluoroscopic image directly after injecting (e.g., 0 seconds after injecting) an area with a radiopaque substance.
Figure 9 shows an aspect of the method carried out by the system according to an embodiment of the present invention, illustrating a fluoroscopic image of an area 30 seconds after being injected with a radiopaque substance (e.g., the image appears blurred).
Figures 10A, 10B, and 10C show aspects of the method carried out by the system according to an embodiment of the present invention, illustrating navigating through at least one bronchus and/or different bronchi, and recording a fluoroscopic image of each navigating event.
Figure 11 shows an aspect of the method carried out by the system according to an embodiment of the present invention, illustrating an augmented bronchogram generated/derived from a combination of images (e.g., but not limited to, Figures 10A, 10B, and 10C), where the images contain a visible instrument in, e.g., but not limited to, at least one bronchus.
Figure 12 shows an aspect of the method carried out by the system according to an embodiment of the present invention, illustrating a straight instrument section projected to a fluoroscope image plane.
Figure 13 shows an aspect of the method carried out by the system according to an embodiment of the present invention, illustrating recovery of depth information related to an anatomical path (e.g., a bronchus/i).
Figure 14 shows a navigation catheter having an anchor (e.g., disposable or non-disposable catheter) for use in an aspect of the method carried out by the system according to an embodiment of the present invention.
Figures 15A and 15B are images showing an aspect of the results obtained from using the method carried out by the system according to an embodiment of the present invention. Figure 15A is a first image (e.g., an original image) and Figure 15B is a second image having a highlighted section (e.g., shown in a dashed circle).
Figure 16 is an image showing an aspect of the results obtained from using the method carried out by the system according to an embodiment of the present invention.
Figure 17 shows an aspect of the method carried out by the system according to an embodiment of the present invention, illustrating a 3D bronchial tree.
Figures 18A-C show an aspect of the method carried out by the system according to an embodiment of the present invention, illustrating the resulting images of injecting a foam mixture into a lung model.
Figures 19A-E illustrate an aspect of the method carried out by the system according to an embodiment of the present invention, illustrating the resulting images of injecting a foam mixture into a lung model.

### DESCRIPTION

The present invention will be further explained with reference to the attached drawings, wherein like structures are referred to by like numerals throughout the several views. The drawings shown are not necessarily to scale, with emphasis instead generally being placed upon illustrating the principles of the present invention. Further, some features may be exaggerated to show details of particular components.

The figures constitute a part of this specification and include illustrative embodiments or aspects useful for understanding the present invention and illustrate various objects and features thereof. Further, the figures are not necessarily to scale, some features may be exaggerated to show details of particular components. In addition, any measurements, specifications and the like shown in the Figures are intended to be illustrative, and not restrictive. Therefore, specific structural and functional details disclosed herein are not to be interpreted as limiting, but merely as a representative basis for teaching one skilled in the ad to variously employ the present invention.

Among those benefits and improvements that have been disclosed, other objects and advantages of this invention will become apparent from the following description taken in conjunction with the accompanying figures. Detailed embodiments of the present invention are disclosed herein; however, it is to be understood that the disclosed embodiments are merely illustrative of the invention that may be embodied in various forms. In addition, each of the examples given in connection with the various embodiments of the invention which are intended to be illustrative, and not restrictive.

Throughout the specification and claims, the following terms take the meanings explicitly associated herein, unless the context clearly dictates otherwise. The phrases "in one embodiment" and "in some embodiments" as used herein do not necessarily refer to the same embodiment(s), though it may. Furthermore, the phrases "in another embodiment" and "in some other embodiments" as used herein do not necessarily refer to a different embodiment, although it may. Thus, as described below, various embodiments of the invention may be readily combined, without departing from the scope of the invention.

Moreover, throughout the specification, phrases such as, for example, "In some embodiment, a/the method of the present invention ...", "In some embodiments, use of a/the method of the present invention ...", "In some embodiments, the present invention is a/the method ...", "In some embodiments of the method of the present invention ...", "In some embodiments, a/the method ...", "... a/the method of the present invention ..." and corresponding phrases have to be understood or read as "A/The method carried out by a/the system according to an embodiment of the present invention ...", "Use of a/the method carried out by a/the system according to an embodiment of the present invention ...", "In a/the method carried out by a/the system according to an embodiment of the present invention ..." or "... a/the method carried out by a/the system of an embodiment of the present invention ...".

In addition, as used herein, the term "or" is an inclusive "or" operator, and is equivalent to the term "and/or," unless the context clearly dictates otherwise. The term "based on" is not exclusive and allows for being based on additional factors not described, unless the context clearly dictates otherwise. In addition, throughout the specification, the meaning of "a," "an," and "the" include plural references. The meaning of "in" includes "in" and "on."

As used herein, "coarse registration" refers to a rough alignment of a preoperative and an intraoperative image. In some embodiments shown, coarse registration uses global information and does not take into account local tissue deformation caused by breathing, instrument movement, pose difference between preoperative and intraoperative images, etc.

As used herein, an "element" refers to a unit of anatomy that has a common mechanical characteristic, for example, a mechanical property (e.g., but not limited to, a rigidity of movement, flexibility, strength). In some embodiments, elements can be, but are not limited to, bronchi, vessels, ribs, image patterns, etc.

As used herein, "fine registration" refers to the registration of local tissue (e.g., but not limited to, soft tissue) around an area of interest of a first image (e.g., a preoperative image), which corresponds to an area of a second image (e.g., an intraoperative image). In some embodiments shown, fine registration is a technique/method designed to correct local tissue deformation and/or relative tissue movement (e.g., but not limited to, movement divergence between ribs and lungs during breathing) inside an area of interest, e.g., but not limited to, a local proximity of a tool tip, a pre-marked nodule area, etc. In some embodiments, fine registration further allows for improvement of local registration accuracy over coarse registration in an area of interest, while coarse registration output, such as transformation matrix, projected primitives, output images, etc., are supplied as input for use of the fine registration.

As used herein, "mapping" refers to transferring a plurality of elements from a first image of a first imaging modality to a second image of a second imaging modality. In some embodiments, mapping can include: (1) identifying a plurality of elements of a first image (2) identifying a plurality of elements of a second image, (3) pairing the plurality of elements of the first/second image to a corresponding plurality of elements of a second/first image, (4) registering (i.e., registration) a plurality of elements of the first/second image to corresponding pairs of the plurality of elements of a second/first image. In some embodiments, the registering is performed by fine and/or coarse registration. As a non-limiting example, mapping can include (1) identifying a plurality (e.g., but not limited to, 2, 3, 4, 5, 6, 7, 8, 9, 10, etc., elements) of elements (e.g., bronchi, ribs, etc.) from a first image(e.g., a CT image), (2) identifying a plurality of fluoroscopic elements on the first image (e.g., a CT image) and a plurality of fluoroscopic elements on the second image (e.g., a fluoroscopic image) (3) pairing a subset of the plurality of elements that are corresponding elements (i.e., to bronchi, ribs) on a second image, (4) registering the elements to the corresponding pairs of the elements on the second image, where the mapping results in a representation of the airway of the first image, or any combination thereof. In some embodiments, an image can be derived from a raw image, e.g., but not limited to, a DRR image, an edited image, a processed image, etc.

In some embodiments, although the term "preoperative image" is used to describe the invention it will be apparent to one skilled in the art that the same concept can be applied when the reference image such as CT, MRI or X-Ray Radiograph imaging is acquired intraoperatively. In some embodiments, the method described herein is applicable for the imaging performed with or without contrast medium.

In some embodiments, a method is described that allows using a first imaging modality (such as CT, MRI, etc.) and planning information by generating an augmented image using a second imaging modality, such as, but not limited to, fluoroscopy, digital subtraction angiography (DSA), etc. In some embodiments, the method further includes highlighting an area of interest and/or structures. In some embodiments, the method can include additional imaging and/or planning information, where the additional imaging and/or planning information can be originated/generated from a first imaging modality, and can include superimposing, as non-limiting examples: (i) a first imaging modality for use in obtaining at least one first image of chest; (ii) manual and/or automatic planning of a surgical procedure through defining landmarks, area of interest, incision points, critical structures, bifurcations, anatomical organs, etc.; (iii) at least one second image obtained from second imaging modality, such as, but not limited to, fluoroscopy and/or DSA, and generation of compatible virtual image, such as a digitally reconstructed radiograph (DRR), from a first imaging modality; (iv) a map ("mapping") of planning data to at least one object and/or structure on the compatible virtual image; (v) a registration of at least one second image or video frame from second imaging modality to first image or its portion sourced from first imaging modality; (vi) planning data identified from the compatible virtual image, sourced from first imaging modality to at least one second image from second imaging modality by means of image registration; (vii) planning data mapped from the compatible virtual image, sourced from first imaging modality to at least one second image from second imaging modality by means of image registration; (viii) a highlighted area of interest, e.g., but not limited to, at least one anatomical structure on the at least one second image sourced from second imaging modality to obtain at least one third image, wherein the at least one third image is augmented, or any combination thereof.

In some embodiments, the method further includes superimposing of at least one image or a derivative of the at least one image, a portion of the at least one image or image based planning information sourced from the first imaging modality. In other embodiments, the method further includes navigation and guidance instructions that aid movement of medical instrument. In some embodiments, the method further includes guidance for positioning the second imaging modality, such as use of a fluoroscopic C-Arm, to allow maintaining optimal visibility for an area of interest. In some embodiments, the method further includes tracking of an anatomic structure(s) on subsequent frames from second imaging modality, such as, but not limited to, fluoroscopic video, having substantially the same acquisition parameters, where the acquisition parameters can include, but are not limited to, mode, position, field of view, to result in generating a augmented fluoroscopic image, where the augmented fluoroscopic image is generated by suppression of a static anatomic structure(s) and/or improving signal to noise ratio of underlying soft tissue. In some embodiments, the method includes performing multiphase registration, where at least one static object(s) having small movement(s) (e.g., but not limited to, 2-5 centimeters), such as, e.g. but not limited to ribs, are first registered. In some embodiments, after the static object(s) are first registered, more dynamic objects such as, but not limited to, diaphragm, bronchi, blood vessels, etc. are registered in the following registration iterations. In some embodiments, the method further includes the interfering structures (e.g., any structure that could interfere with an anatomical focus of a procedure (e.g., but not limited to removing ribs from an image focusing on vessels)) being deemphasized.

In some embodiments, the method as described herein allows for the generation of at least one augmented third image, such as, but not limited to, an intraoperative fluoroscopic image, a DSA image, etc., having a highlighted area of interest and/or structures that can include, but is not limited to: (i) using at least two intraoperative images with known relative movement and/or rotation to allow for the grouping of pixels of the at least two intraoperative images according to the movement variation and/or intensity values of the at least two intraoperative images; (ii) performing registration and/or cross-correlation between at least two sequential intraoperative images to reconstruct structures in the area of interest; (iii) differentiating moving and static structures in the area of interest based on user demand; (iv) highlighting anatomical structures an intraoperative image, or any combination thereof.

In some embodiments, the method as described herein further includes using an x-ray radiographic image of a patient's chest, while the x-ray radiographic image can serve as a reference image for enabling an enhancement of at least one anatomical structure on a second image by use of an analogous process, i.e., cross-correlation of the information from radiographic image obtained with different energy levels.

In some embodiments, the present invention is an augmented fluoroscopy device that allows for the generation of at least one augmented fluoroscopy image, where the augmented fluoroscopy device can include, but is not limited to: (i) a video and image processing unit; (ii) a video input card and/or externally connected device configured to input video signal from a fluoroscopic device; (iii) 3D planning input in internal and/or DICOM format; (iv) augmented video signal output, or any combination thereof.

In some embodiments, the device shown herein is integrated within a fluoroscopic device (i.e., as a module) to obtain RAW data as a signal, and includes a RAW data input card. In some embodiments, the device has a RAW data card instead of a video input card. In some embodiments, the present invention is integrated within a Cone-beam CT system.

In some embodiments, a method is shown for highlighting a tissue or an anatomical structure, where the method can include: (i) selecting the volume of interest on the image sourcing from first imaging modality, such as, but not limited to, CT and/or MRI; (ii) acquiring an image from a second imaging modality; (iii) performing coarse registration between a second imaging modality and a first imaging modality to identify the pose of a virtual camera in the second imaging modality correspondent to the one of second imaging modality; (iv) producing at least one pattern from first the imaging modality for the anatomical structure around a volume of interest is produced; (v) identifying a matching pattern in the second imaging modality using a single pattern or multiple patterns produced from the first imaging modality; (vi) highlighting (i.e., enhancing) a matching pattern from the second imaging modality to enhance the anatomy in the volume of interest on third imaging modality, or any combination thereof.

In some embodiments, the method includes finding and suppressing anatomic structures located outside the area of interest.

In some embodiments, a method of object depth calculation is described that includes, but is not limited to: (i) providing parameters of compatible virtual image sourcing from the first imaging modality, (as a non-limiting example, the first imaging modality can be, but is not limited to, DRR - to fluoroscopy); (ii) determining the object size on a virtual image, such as ribs width on DRR at a specific location; (iii) providing the pose and field of view of the second image (as a non-limiting example: a fluoroscopic camera calculated from a calibration process); (iv) calculating the depth (such as, but not limited to, a distance of a specific object or an object area from a fluoroscopic X-ray source) by use of a comparison between (a) the known object sizes sourced from first image (e.g., but not limited to, a CT image) to (b) an object measured on a second image (e.g., but not limited to, fluoroscopic image), or any combination thereof.

In some embodiments, the object size is determined from: (1) a technical specification and/or (2) the measurement on a compatible virtual image, such as, but not limited to, a rigid tool part length and/or width. In some embodiments, the method includes a tool that is designed to allow the calculation of a trajectory as a combination of depth distances from a second imaging modality camera center.

In some embodiments, the invention provides a device that extend visualization capabilities of fluoroscopic imaging modality that is widely used in diagnostic and treatment medical procedures. In some embodiments, the proposed method, called herein "augmented fluoroscopy," allows enhancing visualization of a specific region of interest within the internal structures of the Patient being evaluated in real time. In some embodiments, the method described herein is utilized for soft tissue visualization. In some embodiments, the method allows for a practitioner (e.g., but not limited to, a doctor, a nurse, a specialist, etc.) to have an increased control over the fluoroscopic visualization capabilities in medical procedures (e.g., for use in soft tissue visualization). In some embodiments, use of the method described herein by trainees reduces the learning curve (e.g., but not limited to, decreases training time, decreases miscalculations, etc.).

In some embodiments, the device presented in this invention includes the following functions: signal input, processing, and display capabilities, where the functions can be installed in, e.g., a procedure room. In some embodiments, the invented device is configured to integrate signals from existing imaging equipment to provide an advanced visualization capability(ies). In some embodiments, the present invention is a stand-alone device. In some embodiments, the present invention is at least one module and is integrated inside the current equipment.

In some embodiments, the method described herein includes performing a preoperative planning using preoperative imaging modality such as, but not limited to, a CT scan or a MRI. In some embodiments, the performed preoperative planning can be used to define the area of interest and/or mechanical properties of the tissue that can be enhanced during real-time fluoroscopy. In some embodiments, the method described herein, in addition to enhancement/highlighting of the area of interest on an intraoperative fluoroscopic image, can generate an overlay on an intraoperative fluoroscopic image. In some embodiments, the overlay can include: the location information of internal and external landmarks together with anatomic structures such as lesion and/or resection boundaries, incision points, bronchial airways, blood vessels, etc. In some embodiments, the method includes: (i) performing preoperative planning and (ii) using the preoperative plan during a diagnostic procedure and/or a treatment procedure. In some embodiments, use of the method described herein improves the efficacy and safety of diagnostic and/or treatment procedures.

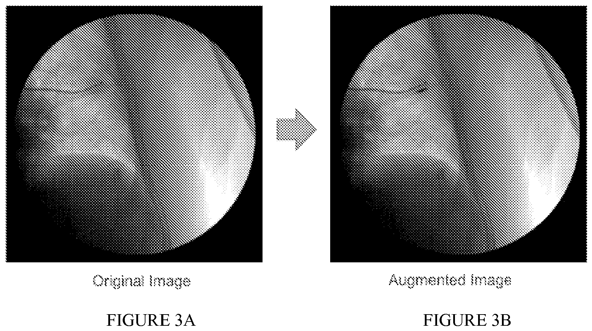

In some embodiments, the present inventions disclosed herein relate to the aspects of augmented fluoroscopy device that allows highlighting the elements or area of interest of the fluoroscopic images in real time. Exemplary embodiments of highlighting include optional superposition (e.g., but not limited to, preoperative planning elements over static or dynamic fluoroscopic images used for diagnostic and/or treatment procedures). In some embodiments of the method described herein, highlighting methods include: (i) bolding a selected area, (ii) coloring a selected area (e.g., selecting an area and placing a pigment (e.g., but not limited to, yellow, blue, red, green, etc.) on a gray scale image, (iii) enhancing an image of a tissue/area (e.g., see Figure 3, where an "augmented image" is an "enhanced image"), (iv) super-positioning a graphic over a fluoroscopic image (e.g., but not limited to, super-positioning a boundary (e.g., a dotted line, a dashed line, etc.) over a selected area of a CT scan), or any combination thereof. In some embodiments, highlighting can be performed automatically, semi-automatically, manually, or any combination thereof.

Conventional fluoroscopy is typically used to obtain real-time moving images of the internal structures of a patient during medical procedures. Conventional fluoroscopy is a visualization and validation imaging tool for guiding medical instruments inside a body (e.g., but not limited to, a human body). Although the bone tissue and medical instruments such as, but not limited to, catheters, biopsy tools, surgical instrument, calibration tool, etc., are clearly visible on a fluoroscopic image, the features of lower density matter such as soft tissue, blood vessels, suspicious nodules etc., are difficult to identify with conventional fluoroscopy. Taking lung cancer diagnostic procedures as an example, a CT scan is usually acquired, prior to procedure. While the pulmonary nodule is clearly observed on the CT scan it cannot be clearly specified on the fluoroscopic image in most of these cases. Prior to a diagnostic and/or a treatment procedure, a health care professional (e.g., a physician) typically studies a preoperative CT scan and/or a MRI image to identify the area of interest that needs to be addressed during an incoming procedure. Using the three-dimensional ("3D") imaging information and professional knowledge/experience, a physician plans the incoming procedure without an actual detailed documentation of such a plan.

During the actual diagnostic or treatment procedure physician is frequently using a fluoroscope to verify/identify the Position and/or Operation of the diagnostic and surgical instrument. Since the target area is not clearly specified on the fluoroscopic image, the physician can be required to guess/estimate the location of the target area. Moreover, since the fluoroscopic image represents accumulated information from the x-rays passing through the Patient, as the x-rays are attenuated by varying amounts when interacting with the different internal structures of the body, the low-density soft tissues are occluded by high-density tissue. In addition, the three-dimensional information is missing from a fluoroscopic image. As a result, there is high probability of user errors caused by misinterpretation of visual information displayed on fluoroscopic images. Finally, the typical approach generally results in a the low diagnostic yield (i.e., the likelihood that a diagnostic procedure will provide the information needed to establish a definitive diagnosis) of 35%, substantially larger resection area margins (e.g., but not limited to, 10%, 20%, 30%, 40%, 50% larger), substantially longer procedure time and inconsistent results within the same medical facility while targeting soft tissue area or nodules through the conventional fluoroscopy.

An electromagnetic navigation system (ENB) may be used in the method of the present invention to support inter-body navigation. The ENB typically uses preoperative static CT images.

The method described herein uses real time fluoroscopic images (i.e., not static images). In some embodiments, the present invention is a device configured to achieve a real time modality that allows a user/practitioner to visualize (effectively) the soft tissue target area of diagnostic and/or treatment procedure with a diagnostic or surgical instrument. In some embodiments, real-time visualization is advantageous, since preoperative static image information, such as CT or MRI, is inaccurate for localization of instruments relatively to the target area due to significant movement and/or deformation of the lung tissue during breathing, where deformation is caused by an advancement of a diagnostic instrument or a surgical instrument inside a patient (e.g., a human body) in addition to potentially substantially dissimilar patient conditions compared between (a) a preoperative CT imaging and (b) actual diagnostic or treatment procedure.

In some embodiments, the method described herein can include use of a third imaging modality configured to use a second imaging modality (e.g., but not limited to, real time fluoroscopy) during a diagnostic treatment or a treatment procedure in conjunction with use of a first imaging modality (e.g., but not limited to, preoperative CT). In some embodiments, the method can include a third imaging modality configured to produce a third image having highlighted elements/features of interest (i.e., augmented image) during a diagnostic and/or a surgical procedure. In some embodiments, the method can facilitate a reduction in operation time and/or an improvement in the learning curve of such procedures (e.g., for a nascent practitioner).

In some embodiments, the method described herein can be used during a surgical procedure and/or guiding under real-time visualization of an area of interest.

In some embodiments, the method allows a practitioner to control visibility of specific elements of an area of interest on a third image (e.g. fluoroscopic image) by adding at least one three-dimensional aspect of information to a second image (e.g. conventional fluoroscopic image). In some embodiments, the method can aid a user to focus on an area of interest (i.e., the correct area of interest required during a surgical procedure), including, for example, an inspection of adjunctive structure around the area of interest, such as, but not limited to, blood vessels, bronchial airways, etc. In some embodiments, the method described herein includes suggesting to a user an optimal fluoroscopic angle to increase visibility of a lesion at the time of a diagnostic and/or treatment procedure, where the suggestion is based on at least one DRR preoperative image.

In some embodiments, the method described herein allows for providing increased control to a physician during a surgical procedure, where the control includes sufficiently improving the physician's ability to accurately identify a treatment area and/or at least one critical structure(s) relatively to the diagnostic instrument and/or surgical instrument according to pre-operative planning and three-dimensional imaging data.

In some embodiments, the method described herein uses a hardware device having integrated software algorithms that are configured to allow for an integration and processing of first images (e.g. pre-procedure) and second images (e.g. intraoperative fluoroscopic), and rendering real-time or offline images of a third image (e.g. augmented fluoroscopy) on an output (i.e., a result).

In some embodiments, the method described herein uses an angular measurement device/sensor (e.g., a right angle sensor, an accelerometer, gyroscope, etc.) that is configured to allow for determining a spatial relative angle and/or position (pose) between: (a) the C-Arm of fluoroscope and (b) the patient.

In some embodiments, the method described herein can utilize a steerable catheter configured to allow measuring a depth inside a patient (e.g., but not limited to, within a patient's chest) and/or a distance from a fluoroscopic camera.

In some embodiments, the device of the present invention provide a real-time third imaging modality (e.g. augmented fluoroscopic modality) to allow for use of (a) information originated from a first image (e.g. pre-operative CT image) and (b) information (e.g., decisions) made during the planning phase for highlighting an area of interest (i.e., providing an augmented image), optionally including a display of (a) the information originated from the first image and/or (b) information generated during the planning phase over second image (e.g. fluoroscopic image).

In some embodiments, the methods described herein can be used to assist the diagnostic and/or treatment procedures involving soft moving tissues such as, but not limited to, lung, liver, kidney, etc. In an exemplary embodiment, in pulmonology, peripheral nodules can be highlighted on a fluoroscopic image and/or a digitally reconstructed radiograph (DRR) image of the peripheral nodules can be superimposed over the fluoroscopic image in real time. In some embodiments, the approach of using three-dimensional CT image to highlight the area of interest on the two-dimensional ("2D") fluoroscopic image is applicable to other medical applications.

In some embodiments, the method described herein can be used with a Cone Beam CT device. In some embodiments, combining the method of the present invention with a Cone Beam CT device allows for greater navigation accuracy, automatic fluoroscopic pose control, radiation dose reduction, etc.

In some embodiments, the method described herein allows a practitioner to navigate and/or operate a medical instrument(s) according to real time information highlighted on third image (e.g. fluoroscopic image/augmented image), where the third image can include superimposed anatomical and/or planning data extracted from a pre-operational image.

In some embodiments, the method described herein provides a real-time third image (e.g. fluoroscopic image/augmented image) of an actual surgical instrument and highlighted area of interest and/or anatomical elements. In some embodiments, the method can provide an overlaid targeted anatomical feature(s) on the augmented image. In some embodiments, the method can provide planning information, such as, but not limited to, incision points, cutting area boundaries, reference points, etc., on the augmented image.

In some embodiments, the device of the present invention allow a user/practitioner to combine multimodal imaging information and utilize previously acquired three-dimensional volume data to highlight moving and static soft tissue area (i.e., generate an augmented image).

In some embodiments, the method described herein includes producing an augmented fluoroscopy image that provides to a user/practitioner an identifying structure(s) on the augmented fluoroscopic image, which is generated by a movement variability analysis of groups of pixels (e.g., different groups of pixels) on a fluoroscopic video and/or sequential fluoroscopic image(s). In an exemplary embodiment, the soft tissue lesion inside the lungs moves in a different direction in comparison with the ribs, and the amplitude of soft tissue movement is typically greater than one of the ribs, resulting in a projected movement of the soft tissue and rib structures having a difference as measured by the fluoroscopic video frames. In some embodiments, the measured difference combined with the information of each pixel attenuation value allows for the grouping of pixels into physical structures and/or objects. In some embodiments, when grouped into objects, the physical structures can be highlighted or deemphasized on the fluoroscopic image in reference to a medical application determined by a user/practitioner. In some embodiments, the augmented fluoroscopic image can be further enhanced by extracting the object information from the sequence of fluoroscopic images, which can be optionally refined with the information provided by a preoperative image such as, but not limited to, CT, MRI, chest x-ray radiographic image, or any combination thereof.

In some embodiments, the method described herein includes an automatic calibration of at least one static fluoroscopic image and/or video frame from a real time video. In another embodiment, the method includes (i) generating a prediction of the quality of specific anatomical structure or visibility of an area of interest during intraoperative fluoroscopy at various angles and (ii) recommending angles to use a fluoroscopic C-Arm for improving visibility of the specific anatomical structure or area of interest, which provides guidance to a user and achieves increased visibility of the structure/area of interest, e.g., relative to the background of an image.

In some embodiments, the method described herein provides processing the RAW data obtained from a fluoroscopic device by changing an existing automatic gain algorithm integrated with the fluoroscopic device, based on the whole fluoroscopic image. In some embodiments, the method includes the use of a region-based gain calculation algorithm. In some embodiments, a specified region-based gain calculation algorithm is derived from the knowledge of correspondent three-dimensional anatomy, where the correspondent three-dimensional anatomy is obtained from CT or MRI images, around the area of interest and includes evaluating the physical properties of the area of interest. In some embodiments, the method provides for a specific signal processing, which reduces a loss of information provided on the resulting fluoroscopic image in the target area (i.e., augmented image), and can also resulting in an increase of visibility of the target area.

In some embodiments, the device of the present invention can be used to maintain/generate an accurate registration (i.e., coarse registration and/or fine registration) between two or more operative real-time video images and/or static preoperative images.

In some embodiments, the device of the present invention can include the use of pre-operative data (i.e., decisions/information generated by a user/practitioner), where information is displayed on the screen, and the resolution and/or quality of the displayed information can be dynamically determined on an application-specific or user-specific basis.

In some embodiments, a method is described that uses a hardware device having integrated software algorithms configured to provide an input from a first imaging modality (e.g. pre-procedure image) and second imaging modality (e.g. intra-operative fluoroscopic image) that generates third imaging modality images (e.g. augmented fluoroscopic image) as output.

In some embodiments, the method described herein provides a real-time output calibrated image with configurable display elements and output video format.

In some embodiments, the method described herein can use a hardware device with integrated software algorithms that has standalone and/or modular architecture.

In some embodiments, the method described herein uses a hardware device that is configured to provide an angular measurement determining relative spatial pose between the fluoroscope C-Arm and Patient body to a user. In some embodiments, the device is applicable for those fluoroscope models where the angular information is unavailable or inaccessible during procedure.

In another embodiment, the method described herein can include reconstructing at least one anatomical structure in a three-dimensional space from several fluoroscopic images (e.g., 2 images, 3 images, 4 images, 5 images, 6 images, 7 images, 8 images, 9 images, 10 images, etc.) by using the correspondent three-dimensional anatomical structures derived from preoperative images, e.g., CT scans ).

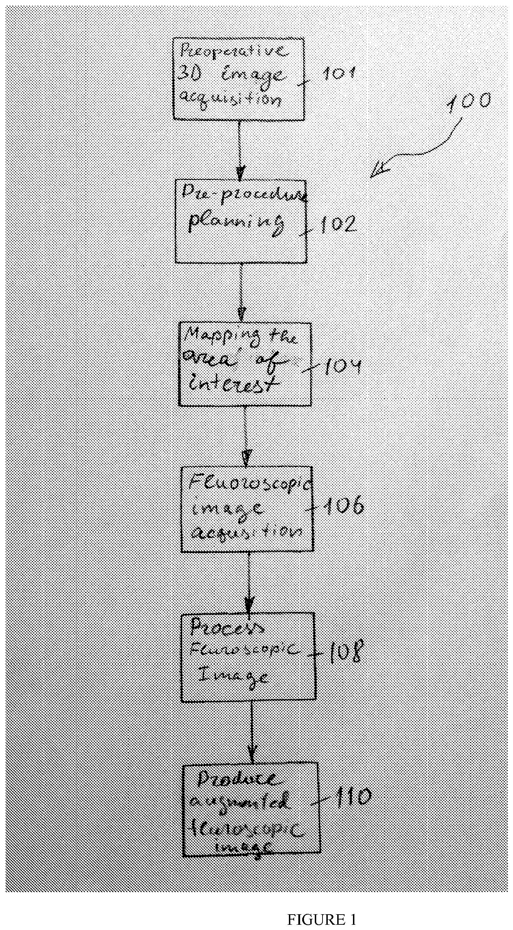

Referencing **Figure 1** there is shown a flowchart that illustrates method **100** of an embodiment.

At **101** of the method **100** first image (e.g. preoperative image, such as CT or MRI), is acquired and transformed into 3D space, which is used during surgical treatment or diagnostic procedure to plan the treatment and/or diagnosis.

At **102** of the method **100** the practitioner (for example, but not limited to, pulmonologist or surgeon) performs pre-procedure planning on the pre-procedure data acquired at **101,** during which the practitioner marks the area of interest (e.g., the boundaries of the area to biopsy or resect around the suspicious lesion, the approach or incision points for preferred tool introduction, critical structures (e.g., but not limited to, major blood vessels, restricted area)), the preferred pathway to approach the area of interest. In some embodiments, the procedure (i.e., **102)** may be performed manually and/or semi-automatically, such as when part of information is automatically identified by computer software.

In some embodiments once the planning is completed, at **104** the information is processed to map (i.e., "mapping") and/or identify (i.e., "identifying") the area of interest, where mapping and/or identifying allows for planning elements in a 3D space and/or identify major anatomical structures. In some embodiments, information gathered from mapping (i.e., "mapping information") is transferred from (a) image sourcing from a first imaging modality to (b) an image sourcing from a second imaging modality. In some embodiments, the mapping information is transferred after the coarse and/or fine registrations are performed on the first image source and the second image source. In some embodiments, an image source (e.g., but not limited to, a first image source) can be use/reused for highlighting purposes during second imaging modality operation (e.g., but not limited to, intraoperative fluoroscopy).

Non-limiting examples of mapping or identifying techniques for body organs are disclosed in "Automatic localization of solid organs on 3D CT images by a collaborative majority voting decision based on ensemble learning" by Zhou X,, Fujita H, Comput Med Imaging Graph. 2012. For example, a location of a target organ in a 3D CT scan can be presented as a 3D rectangle that bounds the organ region tightly and accurately (e.g., serving as a boundary for at least one organ). For example, the location of a target organ-specific 3D rectangle (e.g., but not limited, to a bound rectangle) is detected automatically. Multiple 2D detectors are trained using ensemble learning and the outputs of the multiple 2D detectors are combined using a collaborative majority voting in 3D to localize an organ(s). For example, the location detection of different inner organs can be used separately and/or independently. The exemplary method includes treating 3D organ localization in a 3D CT scan as detecting several independent 2D objects in a series of 2D image slices, where the method can (i) reduce the feature dimension (3D to 2D) and (ii) increase the number of training samples (e.g., one 3D training sample consists of a large number of 2D training samples) during ensemble learning. The exemplary method can increase the robustness of the trained detector for unknown samples according to Occam's razor. For example, for an unknown 3D CT scan, the exemplary method applies different 2D detectors to each voxel independently to detect a number of 2D candidates of a target along three orthogonal directions and votes those 2D candidates back to the 3D space. The existence and approximate center position of the target can be determined by checking the mutual consent of the responses all 2D detectors and selecting the majority of the range of the related 2D candidates in the 3D voting space as the target location.

Non-limiting examples of mapping or identifying techniques for body organs are also disclosed in "Registration of a CT-like atlas to fluoroscopic X-ray images using intensity correspondences," M. Sc thesis by Aviv Hurvitz, supervised by Prof. Leo Joskowicz, The Rachel and Selim Benin (School of Computer Science and Engineering The Hebrew University of Jerusalem, Israel, August, 2008). This exemplary method allows for intraoperative localization of bones, where the method does not require any preoperative images, and is less invasive than many alternatives. For example, in the preoperative stage, a CT-like intensity atlas of the anatomy of interest is constructed from sample CT images. In the intraoperative stage, a novel 2D/3D deformable registration algorithm is used to register the atlas to Fluoroscopic X-ray images of the patient anatomy. The registration algorithm is configured to establish intensity-based correspondences between the atlas's template bone surface and bone contours in the fluoroscopic X-ray images. The registration algorithm further is configured to search for the bone shape and pose that minimize/reduce the distances between paired features. The algorithm iteratively is configured to refine the bone shape and pose estimates until the bone shape and the pose estimate(s) converge.

In some embodiments, the method includes generating an augmented 3D fluoroscopic image by use of a 2D fluoroscopic image by matching each pixel on the 2D fluoroscopic image to 3D structures sourced from a CT scan. The method described herein does not utilize tracing elements and/or markers, such as, but not limited, to radiopaque marker tethered to a device, a radiopaque particulate spray, an inflatable radiopaque balloon, a radiopaque filament, during a registration.

In embodiments, the method described herein can generate: (i) visualization data that shall be displayed during surgical procedure; (ii) a recommended pathway for introduction of at least one medical instrument; (iii) guidance instructions based on anatomic knowledge and procedure details; (iv) recommended angles or pose for C-Arm, as to result in optimizing the area of interest visibility, or any combination thereof.

In some embodiments, the fluoroscopic image is acquired at **106** during procedure while medical instrument is introduced into the area of interest. In some embodiments, the fluoroscopic image can be acquired as single image and/or video.

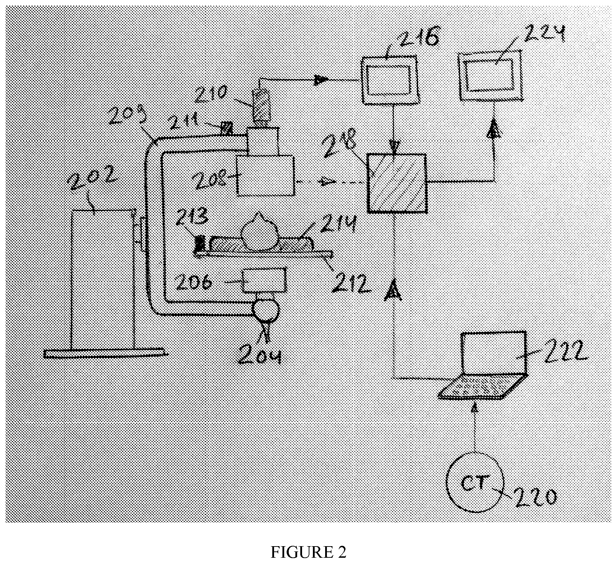

In an embodiment, the generated fluoroscopic image and/or video is introduced into the processing unit **218,** **Fig 2** as an input for fluoroscopic image processing **108.** In the embodiment, the pose between the Fluoroscopic C-Arm **209,** **Fig 2** and patient **214,** **Fig 2** is either transmitted from outside or calculated by processing unit. In the embodiment, the compatible digital reconstructed radiograph (DRR) image is generated from a pre-procedure image using substantially the same pose of a virtual C-Arm and substantially the same camera parameters as the actual Fluoroscope. In some embodiments, the image is calibrated, where "calibrated" means being adjusted for fluoroscopic image distortion and compensated for x-ray energy difference between the fluoroscope and CT at the intensity values according to the Prior art knowledge of X-ray radiometry.

In some embodiments, the following references discuss DRR simulation, calibration and registration to actual fluoroscopic images: "2D/3D Image Registration on the GPU," Alexander Kubias, University of Koblenz-Landau, Koblenz, Germany, Thomas Brunner, Siemens Medical Solutions, Forchheim, Germany, 2007. For example, this exemplary method performs the rigid 2D/3D image registration efficiently on the GPU [graphics processing unit]. Both parts of the registration algorithm, i.e. the DRR generation and the computation of the similarity measure, are executed on the GPU. Additionally, reference is made to "2D/3D Registration for X-ray Guided Bronchoscopy using Distance Map Classification," by Di Xu, Sheng Xu, Daniel A. Herzka, Rex C. Yung, Martin Bergtholdt, Luis F. Guti&rez, Elliot R. McVeigh. For example, the registration algorithms can be grouped into two categories: (1) intensity based and (2) feature based, where the feature-based registration can be used in connection with the method of the present invention. For example, the edges of the ribs and seine can be extracted from the X-ray and/or CT images. A distance map can further be generated for a plurality of (e.g., but not limited to, each recorded edge point, which can result in using all edge points) the edge points of the X-ray image to facilitate/allow the 2D/3D registration by attracting the edge projections of the CT image to the closest edges in the X-ray image. When the distance map does not have any orientation information of the edges, mis-registration can occur between the edges of different structures. Mis-registration can be reduced by using orientation dependent distance maps to achieve more robust registration with improved capture range and accuracy.

In some embodiments, the map generated in **104** is used to provide spatial information for each projected element on the DRR image. In some embodiments, the registration is performed between DRR and actual fluoroscopic images. Examples of registration, e.g., feature-based or intensity-based registration, are described in "Automatic registration of portal images and volumetric CT for patient positioning in radiation therapy", (See, e.g., Ali Khamene, Frank Sauer, Medical Image Analysis 10 (2006) 96-112). For example, the feature based registration approach can involve a step of feature correspondence between features of each of the imaging modalities participating in registration process. As a result of the registration the spatial information generated for DRR image can be transferred onto the actual fluoroscopic image. The 3D spatial information added to the actual fluoroscopic image allows implementing computer vision approach to the actual fluoroscopic image, thus operating with objects in 3D space rather than working with 2D image of Pixels. Using this approach allows for each pixel of a fluoroscopic image to be described by integration of X-ray beam passing through known anatomic structures.

In some embodiments, the information that was lost during fluoroscopic image acquisition is restored using the method of the present invention. In some embodiments, the area of interest can be highlighted on the actual fluoroscopic image, while the interfering structures such as bones, heart, blood vessels can be deemphasized. In some embodiments, an additional improvement of the augmented image quality can be achieved through the tracking of sequential video frames, where the movement characteristics may vary for different anatomic structures.

The augmented fluoroscopic image or video frame sequence is produced in **110** using an embodiment of the method described herein In some embodiments, various elements generated on the planning phase can be displayed on augmented fluoroscopic image according to user demand or depending on system configuration.

**Figure 2** shows a diagram illustrating an embodiment of the present invention, showing an augmented fluoroscopy system/method and data flow.

In an embodiment described herein for producing an augmented fluoroscopic image, the method included use of:
1) C-Arm **202** that is responsible for movement of frame **209** with attached fluoroscopic pair of X-Ray tube **204** and intensifier **208;**
2) X-Ray tube **204** that generates X-rays, passing through the collimator **206,** that is designed to narrow the X-ray beams;
3) the generated X-ray beam is passing through the patient body **214** attached to the bed **212;**
4) the attenuated X-Ray beam is further absorbed by X-ray image intensifier **208** forming the RAW data fluoroscopic image. The X-ray is converted into the visible image by **208;** and/or
5) the video signal is constantly captured by camera **210** and transferred to the monitor **216.**
6) a planning station 222 that is getting CT image **220** as an input allows user to plan diagnostic and treatment procedure as specified by **102, 104** **Fig 1** above;
7) a generated planning data, 3D volume data are transferred into unit **218,** where a video signal from **216** or alternatively RAW data from **208** is constantly transferred to the processing unit **218;**
8) the augmented video image is produced by **218** as specified by **108, 110** **Fig 1** and displayed by the monitor **224;**
9) or any combination thereof.

In an embodiment of the present invention, the following elements were added to provide the C-Arm pose measurement: (1) a sensor **211** attached to frame 209 of C-Arm and/or (2) a reference sensor **213** attached to the patient body **214** and/or to patient bed **212.**

Examples of sensing technologies available for use in embodiments of the present invention to allow for evaluation of pose estimation can include: an optical sensor, an accelerometer, an electro-magnetic sensor, an ultra-sonic sensor, a gyroscopic sensor (e.g., available on the modern smart phones), etc. An example of use of a pose estimation approach, which can be used in the method of the present invention, is described in "Robust Multi Sensor Pose Estimation for Medical Applications" by Andreas Tobergte, Gerd Hirzinger, Intelligent Robots and Systems, 2009. IROS 2009. IEEE/RSJ International Conference.

In some embodiments, the method can use a set(s) of markers with predefined geometric configuration can be attached to the Patient bed as discussed in "Fast Marker Based C-Arm Pose Estimation" by Bernhard Kainz, Markus Grabner, and Matthias Rulher, Institute for Computer Graphics and Vision, Graz University of Technology, Austria.

**Figure 3** shows an exemplary embodiment of the present invention, showing an illustration of an augmented fluoroscopic image. In an embodiment, the diagnostic instrument and bones are clearly seen on the original image while the target area is invisible or unclear. In an embodiment, the target area is highlighted on the augmented fluoroscopic image, e.g., on the right. In an embodiment, the method includes highlighting blood vessels, while deemphasizing the bones.

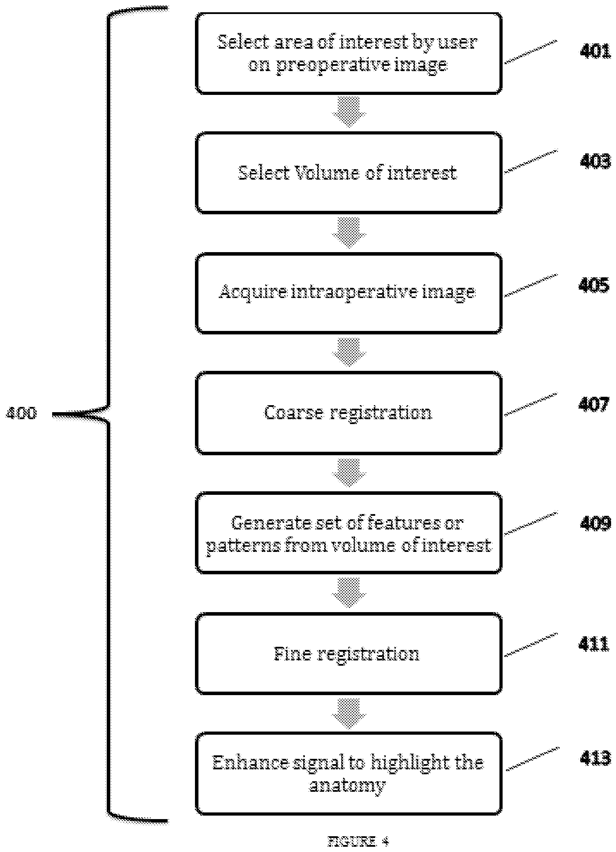

**Figure 4** shows an embodiment of a method that can be used, showing a flowchart of the method **400.** At **401** of the method **400** shows an area of interest being selected by user on preoperative image, such as CT or MRI prior to diagnostic or treatment procedure. At **403** of the method **400,** the volume of interest is generated on preoperative image. In an embodiment, the volume is generated in such way that the anatomical structures in the area of interest, such as lesion, and adjunctive anatomical structures such as bronchi or blood vessels, will be detectable on operative image, such as fluoroscopic image. In an exemplary embodiment, for instance, DRR image can be used to evaluate detectability on fluoroscopic image.

In some embodiments of the method described herein, at **405** of method **400,** intraoperative image or videos are acquired. In an embodiment, the pose of the intraoperative modality is calculated or recorded with at least one intraoperative image. In an embodiment, at **407** of the method **400,** the coarse registration between intraoperative and preoperative images is performed, e.g., but not limited to, fluoroscopy to DRR, to evaluate a viewpoint of DRR inside a preoperative image data, such as, but not limited to, CT volume. An example of coarse registration is shown in "2D/3D Image Registration on the GPU," by Alexander Kubias, University of Koblenz-Landau, Koblenz, Germany, Thomas Brunner, Siemens Medical Solutions, Forchheim, Germany, 2007. Some embodiments of the method described herein use, for example, a rib-based rigid image registration: For example, using 2D/3D image registration, a preoperative volume (e.g. CT or MRT) is registered with an intraoperative X-ray image. Rigid image registration can be used by the method of the present invention, where a volume can only be translated and rotated according to three coordinate axes, where a transformation is given by the parameter vector x = (tx, ty, tz, rx, ry, rz) . The parameters tx, ty, tz represent the translation in millimeters (mm) along the X-, Y- and Z-axis, whereas the parameters rx, ry, rz belong to the vector r = (rk, ry, rz). In some embodiments, coarse registration can be performed automatically.

In some embodiments, the method described herein can use the registration techniques disclosed in, "Automatic registration of portal images and volumetric CT for patient positioning in radiation therapy," by Ali Khamene, Frank Sauer, Medical Image Analysis 10 (2006) 96-112. In exemplary embodiments, such registration can be implemented, as a non-limiting example, as intensity-based and/or as feature based, depending on the specific medical application. Examples of intensity-based and feature based registration are described by "Intensity-based Registration versus Feature-based Registration for Neurointerventions" by Robert A., David J. Hawkesb, Medical Vision Laboratory, Dept of Engineering Science, University of Oxford, England.

In some embodiments of the method described herein point-based registration can be implemented using known anatomical landmarks on a patient's chest. In some embodiments, at least one known landmark(s) can be marked on a CT image and/or fluoroscopic image. In some embodiments, special markers can be attached to the patient's chest during procedure to improve/increase detectability on a fluoroscopic image.

In some embodiments, at **409** of the method **400,** the set of features or patterns, depending on desired registration method, is generated from a volume of interest of the preoperative image. In some embodiments, when the soft tissue structures of a patient are observed and move relative to the ribs of the patient, the viewpoint calculated during coarse registration at **407** is approximated within the known tolerance. In some embodiments, the set of patterns generated at **409** will allow performing the fine-tuning (i.e., fine registration) of the viewed area in the following step.

In some embodiments, at **411** of the method **400,** fine registration is implemented to find the best fit between each of the features or patterns, depending on the registration method, generated at **409** and area of interest on intraoperative image.

In an exemplary embodiment, a fine registration method is illustrated through intensity-based fine registration (i.e., template matching), e.g., as shown in **Figure 5****,** where the approach is initiated with an intensity-based pattern, as shown in **Figure 5A****,** from a pre-operative or a reference imaging modality. In an embodiment, the signal from an intraoperative image, as shown in **Figure 5B,** contains noise and scale corresponding to the pattern shown in **Figure 5A****,** and is measured within the area of interest. In an embodiment, the pattern shown in **Figure 5A** is matched to the pattern from signal **Figure 5B.**

An example of a fine registration (i.e., template matching) technique that can be used by the method of the present invention is described in: "An Overview of Template Matching Technique in Image Processing" by T. Mahalakshmi, R. Muthaiah and P. Swaminathan School of Computing, SASTRA University, Thanjavur, Tamil Nadu, India, Research Journal of Applied Sciences, Engineering and Technology 4(24): 5469-5473, 2012. Some embodiments of the method of the present invention use an area-based approach, which are also referred to as correlation-like methods or fine registration (i.e., template matching), see, e.g., Fonseca and Manjunath, "Registration techniques for multisensor remotely sensed imagery" PE & RS-Photogrammetric Engineering & Remote Sensing 62 (9), 1049-1056 (1996), which describes the combination of feature detection and feature matching. For example, this method is suited for the templates which have no strong features corresponding to an image, since the templates operate directly on the bulk of values. Matches are estimated based on the intensity values of both image and template. Techniques that can be used by the method of the present invention include: squared differences in fixed intensities, correction-based methods, optimization methods, mutual information, or any combination thereof. In some embodiments, the method of the present invention can perform a fine registration automatically.

In some embodiments, the method described herein can perform a coarse registration automatically.

In an exemplary embodiment, the method described herein can utilize a fine registration method, where the fine registration method includes aligning a 2D projection of an anatomical structure from a CT scan obtained through coarse registration with correspondent anatomical structure extracted from fluoroscopic image.

At **413** of the method **400** the signal matching Pattern is shown in **Fig 5A****.** Inside the signal **(Fig. 5B)** is enhanced to highlight the anatomy found in the area of interest as drawn by **401.** In some embodiments, in addition to highlighting the signal from intraoperative image, the signal sourcing from reference image can be overlaid on the display/image. In another embodiment, the combination of original signal from intraoperative image, simulated signal from reference image and planning information can be displayed according to application configuration or upon the user request. In some embodiments, the method shown in **Fig. 5C** can be alternatively used for signal suppression.

**Figure 5** shows an illustrative example of fine registration (as shown in step **411** of **Figure 4**) (i.e., template matching) of the method described herein.

Although this illustration is shown in one dimension for simplicity purposes, the original signals of the embodiment are two-dimensional. In some embodiments, steps **411** and **413** of **Figure 4** provide the methods using a template-matching registration approach.

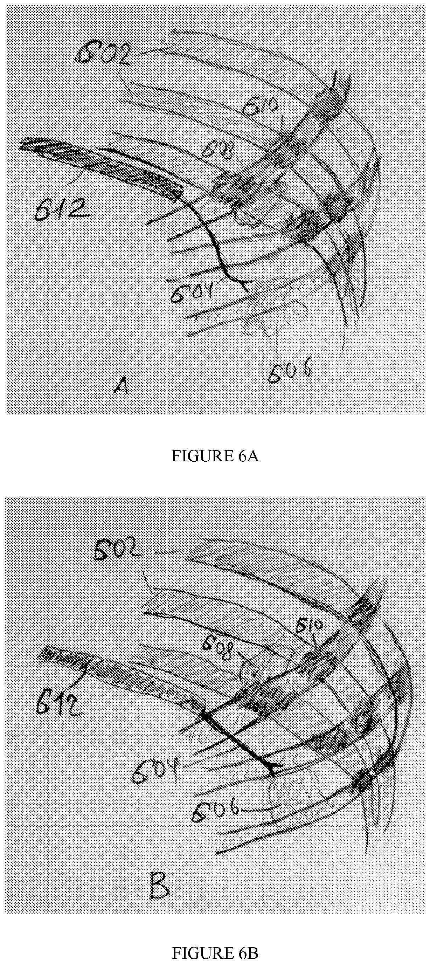

The exemplary embodiment shown in **Figure 6****,** is a schematic drawing of a fluoroscopic image, where **A,** **Fig 6** and **B,** **Fig 6** represent fluoroscopic images for two different lung positions during breathing. In the embodiment, the ribs **602** remain almost static while the soft tissue lesions **606** and **608** move substantially between the two breathing positions. In an embodiment, the tip of the forceps **604** is located in the dose proximity of lesion **606,** which results in the forceps moving with the lesion **606,** while the bronchoscope **612,** which is located far from the lesion, is substantially static and does not substantially move between two breathing positions **A** and **B.** In an embodiment, the rib intersection area **610** is darker then the rib **502** and can be potentially confused with lesion on the conventional fluoroscopic images. In some embodiments, the analysis of sequential fluoroscopic images **A** and **B** allows to separate substantially static and moving objects, group the static and moving objects by (i) movement, (ii) connectivity, (iii) density, or any combination thereof, and/or perform reconstruction of anatomic structures from a plurality of fluoroscopic images.

In some embodiments, the method can be used for the following pulmonology-based procedures including, but are not limited to:
1) Endobronchial diagnostic biopsy, when the pulmonologist first identifies the lesion under augmented imaging. Then, the biopsy forceps are advanced to the target site under augmented imaging to insure the biopsy is taken appropriately;
2) Augmented imaging guided percutaneous diagnostic biopsy;
3) Wedge resection with VATS or thoracotomy when thoracic surgeon places markers augmented fluoroscopy guidance prior to surgical procedure;
4) Trans-bronchial needle biopsy direct vision is used to visualize the lesion and to guide the bronchoscope. The area to be biopsied is first identified under augmented imaging and then the scope is advanced as far as possible to the targeted segment. Using augmented imaging helps to guide the forceps distally to the target area, beyond the range of direct vision;
5) Augmented imaging guided endobronchial or percutaneous ablation;
6) Or any combination thereof.

In some embodiments, the present invention is used to generate multidimensional images from 2D fluoroscopic images. In some embodiments, a 2D fluoroscopic image is displayed in gray levels and comprised of pixels. In some embodiments, each pixel represents an integrated density of at least one tissue while an x-ray generated by an x-ray tube is absorbed by an image intensifier.

In some embodiments, the objects of higher density (e.g., bones and blood vessels) have greater weight on the integrated pixel density (color) in comparison with integrated pixel density of, e.g., air and/or soft tissue. In some embodiments, automatic gain algorithms implemented for fluoroscopic devices make at least one high-density tissue visible while reducing the visibility of at least one soft tissue. In some embodiments, at least one suspicious lesion area, although having small volume relative to, e.g., bones, has higher tissue density than at least one normal tissue. In some embodiments, at least one suspicious lesion area is characterized by increased blood activity (e.g., flow and/or volume) in comparison to at least one area around normal tissue. In some embodiments, at least one natural anatomic characteristic of a suspicious lesion area (e.g., in soft or dense tissue), includes at least one shadow and/or cloud-like object observed by at least one fluoroscopic image. In some embodiments, there are additional sources for the at least one shadow and/or cloud-like object by at least one fluoroscopic image (e.g., at least one rib cross-section, joint, major blood vessel, etc.)

In some embodiments, a method is described that separates at least two different (e.g., non-identical) portions of visible tissue(s) (which can be the same or different tissue) on a fluoroscopic image and combines the at least two different portions into objects through segmentation and tracking of visible tissues using optical flow on fluoroscopic video. In some embodiments, the Pixels on a fluoroscopic screen are (1) classified by density range, (2) tracked through the live fluoroscopic video, and (3) classified by movement. For example, breathing includes lung expansion and contraction movements, which vary from lobe to lobe in the same lung and also vary from movement of ribs. Such movements result in a lung projection, and can be shown by the fluoroscopic video images generated from the method described herein, characterized by a plurality (e.g., a variety) of movements for every distinguishable anatomical structure as illustrated by Figure 6.

In some embodiments, the method described herein includes a registering process/step, where the registering process/step uses as input: a segmentation of bronchial airways from (i) a fluoroscopic image and (ii) a CT scan. In some embodiments, a course and/or fine registration is performed using a registering step.

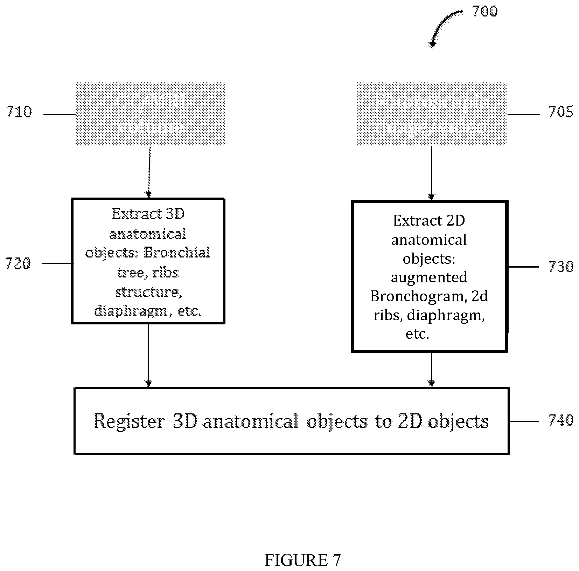

In some embodiments, a method allows registration between at least one bronchial airway tree extracted from a preoperative CT image and airways extracted from fluoroscopic image sequence using augmented bronchogram. In an embodiment, a general flow is illustrated in **Figure 7****.**

In some embodiments, the present invention provides an augmented bronchogram. In some embodiments, the augmented bronchogram is an augmented image of invisible airways (e.g., not visible by fluoroscopic image) and is extracted from fluoroscopic images.

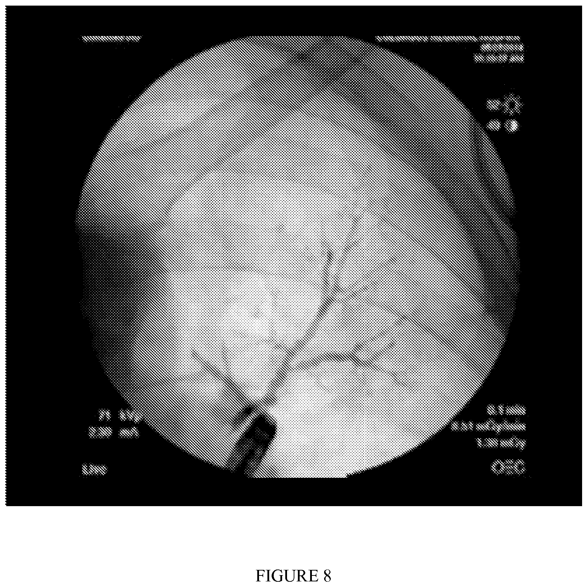

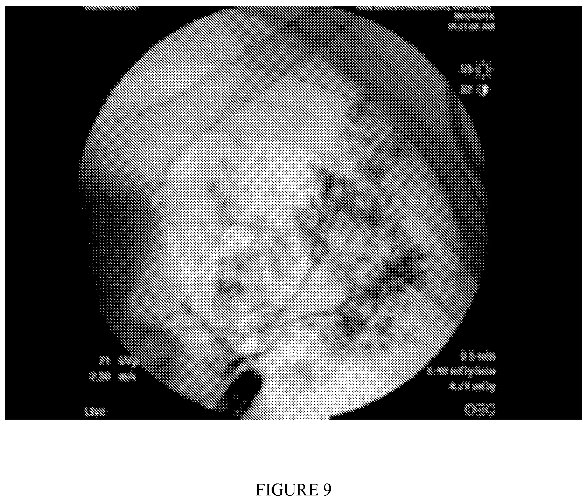

In an embodiment, an augmented bronchogram is generated by injecting a radiopaque substance configured to make bronchi visible **(****Figure 8****).** In an embodiment, visible bronchi provide information (1) to extract a partial bronchial tree from fluoroscopic images and (2) to register the partial bronchial tree to a second image, e.g., the bronchial tree extracted from a preoperative image. In some embodiments the radiopaque substance injected in bronchi does not highlight (i.e., make visible) the airways uniformly. In some embodiments, the radiopaque substance quickly disappears from an image or disperses (e.g., but not limited to, within 1 - 60 seconds, 1 - 45 seconds, 1-30 seconds, 1-15 seconds, etc.), which deteriorates fluoroscopic image quality **(****Figure 9****),** and creates a blurred image. In some embodiments of the present invention, at least one image processing algorithm is utilized to generate a bronchogram. In some embodiments of the present invention, at least one image processing algorithm is utilized to generate an augmented bronchogram.

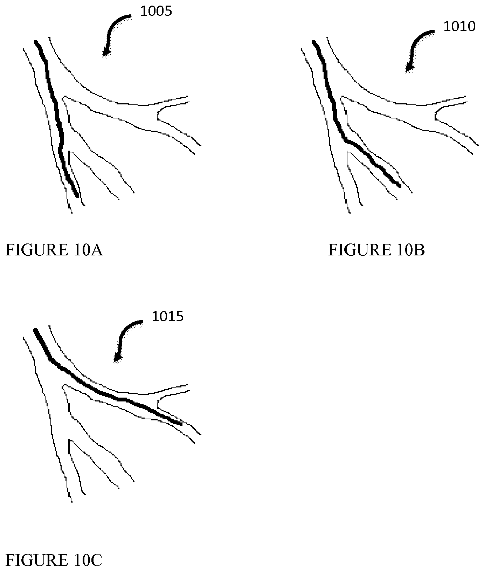

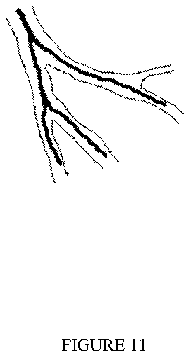

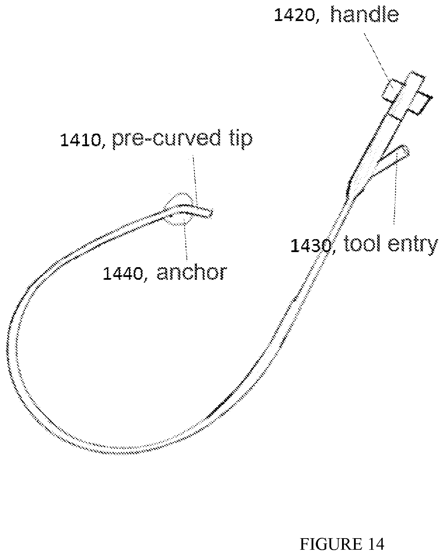

In some embodiments, an augmented bronchogram is created by using at least one radiopaque instrument, that has can optionally have anchoring mechanism as drawn by **Figure 14****.** In some embodiments, the radioscopic instrument is visible in fluoroscopic images and represents an anatomical structure that can be registered to the bronchial tree, which is identified from at least one preoperative image. In some embodiments, the direct extension of this method is using multiple instrument positions **(****Figure 10****)** extracted and accumulated from temporal fluoroscopic image sequence during the same procedure **(****Figure 11****).** In some embodiments, the radiopaque instrument can be multi-lumen, where lumens can be used for: (i) diagnostic or treatment procedure, (ii) introducing multiple radiopaque guide-wires simultaneously into multiple bronchial airways and using the guide-wires as a plurality of registration references. In some embodiments, this technique improves registration accuracy and robustness.

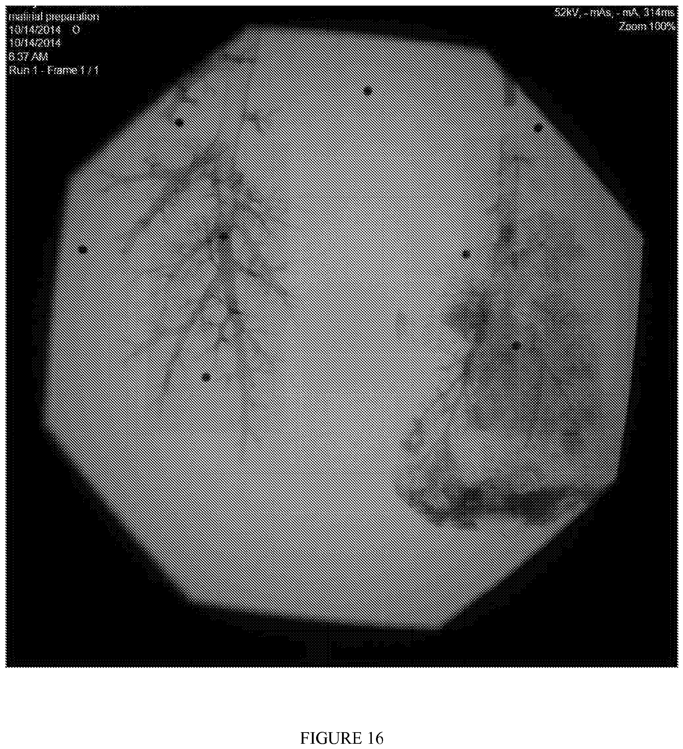

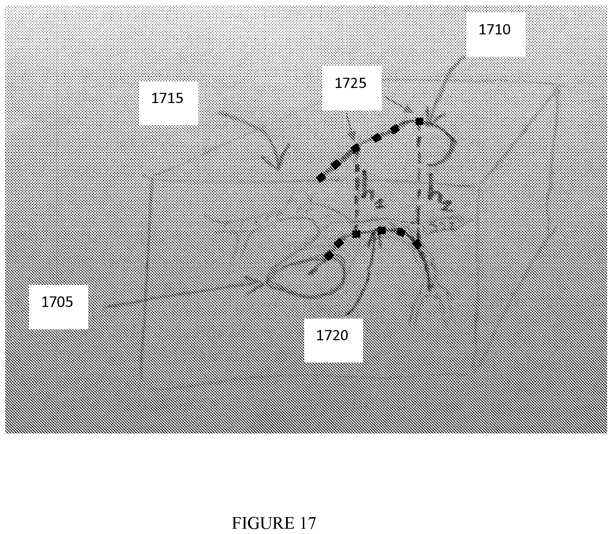

In some embodiments, an augmented bronchogram is created using at least one instrument that allows perfusion of radiopaque substance to remain visible and in place (e.g., substantially static) for an increased period of time. In some embodiments, the increased period of time is achieved by using the at least one instrument that spreads at least one radiopaque substance on the walls of airways using a brush or sprinkles on the tool exterior. In some embodiments, a radiopaque substance having a high viscosity (e.g., in the form of hydrogel) is injected through the instrument and dispersed on the airways. In some embodiments, the radiopaque material is configured to be gradually released from the radiopaque substance. In some embodiments, the airway area retains a radiopaque characteristic for longer period of time. In some embodiments, a reverse thermo-gelling polymer or similar material is used, to allow effective injection of liquid substance at a low temperature while prevention of fluoroscopic image quality deterioration **(****Figure 9****)** or blurred fluoroscopic image since the injected substance becomes a semisolid gel as the temperature increases to the body temperature. In some embodiments, the reverse thermo-gelling (e.g., radiopaque) substance is used to generate a foam-based colloid (e.g., but not limited to, AculynTM22 (A22) and AculynTM33 (A33), can be emulsions used to generate a foam, Bepanthen^{®}, post-expansile foam aerosol of Propylene Glycol-liposomes (PEHFL), polyurethane-based shape-memory polymer (SMP) foams combined with cold hibernated elastic memory (CHEM), or any combination thereof), which the foam-based colloid is injected inside the area of interest of the lung and configured to remain stable during a predefined or sufficient period of time (e.g., a period of time which allows for a health care professional to ascertain results prior to image quality deterioration; e.g., 30 seconds, 1 minute, 5 minutes, 10 minutes, 15 minutes, 30 minutes, etc.). For example, as it is shown on the left side of **Figure 16****,** the foam based-radiopaque substance is configured to highlight the lung for a long period time (e.g., over 30 minutes) while preventing fluoroscopic image quality deterioration (e.g., Figure 9, Figure 10 on the right) that typically occurs within a few seconds (e.g., 1 second, 2 seconds, 3 seconds, 5 seconds, 10 seconds, 1 minute, etc.) after conventional contrast injection. In some embodiments, an instrument can be designed to include multiple radio opaque markers 1725 that are placed in predetermined locations in order to reconstruct the depth of the instrument from single 2D projection of the instrument on fluoroscopic image **(****Figure 17****).**

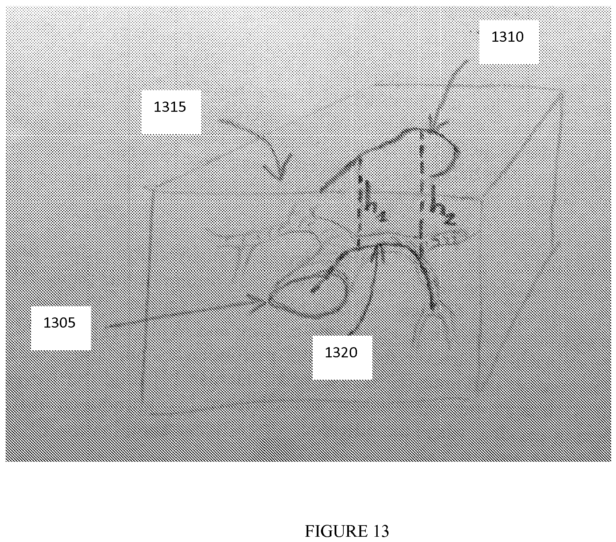

In some embodiments, a method is described that includes adding a third dimension (depth) to a position of an instrument on a 2D fluoroscopic image. In some embodiments, a depth of at least one section of the instrument is calculated by (1) comparison of (a) the projected instrument shape on fluoroscopic image with (b) the known anatomical structure of the bronchial airway and (2) making an assumption of constrained instrument location inside the bronchial tree **(****Figure 13****).**

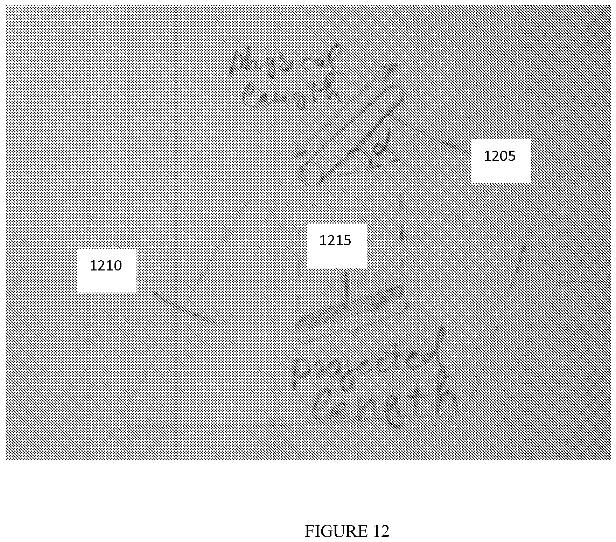

In some embodiments, a method is described that includes adding elevation of the instrument (orientation angle) in a direction perpendicular to a fluoroscopic image. In some embodiments, there are at least two methods to calculate orientation magnitude: (1) comparing the projected and actual physical lengths of a radiopaque straight instrument section, which uses a known zoom (i.e., magnification) of the fluoroscopic image (e.g., from an available registration) **(****Figure 12****),** and (2) using an orientation sensor attached to the instrument to calculate the orientation of the instrument relative to the body of a patient or relative to the fluoroscopic device.

In some embodiments, a method is described includes integrating information including 3D location and orientation to determine the 6 degrees of freedom (DOF) of the instrument inside the patient (e.g., a human body).

In some embodiments, a method is described to track motion and orientation of a tip of an instrument using integrated sensors located on the tip. In some embodiments, the sensor is selected from a group consisting of: a gyroscope, an accelerometer and/or a magnetometer. In some embodiments, the transmitted information from these sensors allows calculating the orientation and the location of the tip in real time. In some embodiments of the present invention, the robustness of the location calculation is improved (i.e., increased accuracy) by assuming/predicting the samples are inside the bronchi. In some embodiments, the samples are registered to the 3D bronchial tree extracted from the preoperative CT image.

**Figure 7** is a flow chart illustrating method **700.** In some embodiments, the flow chart presents the registration process between bronchial airway tree extracted from preoperative image (e.g., but not limited to, a CT scan/image) and airways extracted from fluoroscopic images (e.g., 2, 3, 4, 5, 6, 7, 8, 9, 10, etc.) using an augmented bronchogram. In some embodiments, **710** of the method **700,** a CT and/or MRI is a source preoperative and/or intraoperative image. In some embodiments, the preoperative and/or intraoperative image is acquired and transformed into 3D space, and used during surgical treatment and/or diagnostic procedure for a treatment and/or a diagnosis. In an exemplary embodiment, at **720** of the method **700,** a 3D bronchial tree is extracted from the image **710** using (1) an automatic segmentation algorithm and/or (2) a manual notation by a physician. In an exemplary embodiment, at **705** of the method **700,** there is a source fluoroscopic image and/or fluoroscopic video captured from the fluoroscope. In an exemplary embodiment, at **730** of the method **700,** an augmented bronchogram is calculated using fluoroscopic image **705** by one or more approaches disclosed in the present invention.

In some embodiments, the method described herein includes an automatic separation/segmentation between soft tissue, bones, instrument(s), an anatomical object(s), and background, where the automatic separation/segmentation uses instrument and/or tissue movement to differentiate between different types of tissues/organs and/or instruments (e.g., movement and/or density) to result in the generation of extracted information (e.g., a bronchial tree).

In an exemplary embodiment, the 3D bronchial tree, and optionally 3D structures such as ribs, body boundary, diaphragm, etc., extracted by **720** and augmented bronchogram and optional correspondent structures, such as ribs, body boundary, diaphragm, etc. extracted by **730,** are registered at **740** using the method shown in **700.** In an exemplary embodiment, the registration process estimates pose information (e.g., position, orientation, and/or camera parameters) of the fluoroscope that would project a 3D bronchial tree to match a 2D augmented bronchogram, and produces a correspondence between 3D space of the image **710** and 2D space of the image **705.** Moreover, assuming that the third dimension (depth) information of 2D bronchogram is available prior the registration process, the registration accuracy can be enhanced by using this additional embodiment. For example, this prior information (e.g., the third dimension information of the 2D bronchogram) can be used to reduce the degrees of freedom in the pose estimation process.

In an embodiment, **Figure 8** shows a sample of augmented bronchogram obtained from a sequence of fluoroscopic images containing an injected radiopaque substance that highlights a partial bronchial tree.

In an embodiment, **Figure 9** shows a fluoroscopic image, which is the same subject as in **Figure 8****,** but the image was taken after 30 seconds of injection. As shown, the injected radiopaque substance diffuses to the surrounding regions, producing a blurred image. In an embodiment, an augmented bronchogram produces a clear image after 30 seconds of injection.

**Figure 10** shows an illustration of the method of use of a radiopaque instrument that is visible on fluoroscopic images. In an embodiment, the images, e.g., **1005, 1010** and **1015,** show fluoroscopic views containing a visible instrument in different locations and a schematic structure of a bronchial tree that is not visible in a real fluoroscopic image, and shown here for illustration purposes only. The instrument shown in views **1005, 1010** and 1015 can be the same instrument or different instruments.

In an example, superposition of imaging incorporates distortion correction caused by body movement, breathing, instrument introduction, etc. In some embodiments, the temporal instrument positions are acquired for superposition at the predefined breathing phase.

In an exemplary embodiment, **Figure 11** illustrates the augmented bronchogram, derived from the views **1005, 1010** and **1015** from **Figure 10****.** In an embodiment, each view adds information regarding the surrounding anatomical structures. In an embodiment, the information is combined to create an augmented bronchogram.

In an embodiment, **Figure 12** shows a straight section of an instrument **1205,** located in the 3D space inside the body. In an embodiment, the instrument is projected on the fluoroscope image plane **1210** and created the projection image **1215.** In an embodiment, the angle between the straight section of the instrument **1205** and the fluoroscope image plane **1210** is "alpha."

In an embodiment, **Figure 13** shows a 3D bronchial tree **1315,** containing an anatomical path **1320,** located inside the airways. In an embodiment, when the 3D anatomical path **1320** is projected on the fluoroscope image plane **1315,** the projection **1310** loses the original depth information. In an embodiment, the present invention recovers this information.

In an embodiment, **Figure 17** shows a 3D bronchial tree **1715,** containing an anatomical path **1720,** located inside the airways. An instrument with multiple markers of predefined locations along the instrument **1725** is located inside the anatomical path **1720.** In an embodiment, when the 3D anatomical path **1720** is projected on the fluoroscope image plane **1715,** the projection 1710 loses the original depth information. In an embodiment, the present invention recovers this original depth information.

In an embodiment, **Figure 14** shows disposable navigation catheter with anchoring, that can be guided by means of pre-curved tip 1410 through the bronchial airways. The tool handle 1420 can be optionally used to enhance navigation performance. The catheter tip can be fixated inside the bronchial airways by means of anchor 1440 that is designed as inflatable balloon or extendable spring, to allow instant multiple access to the area of interest around the catheter tip by medical instrument. The diagnostic and treatment instrument can be introduced through the working channel located inside the navigation catheter at the entry point 1430. The wire can be placed inside the catheter during maneuvering inside the bronchial airways to prevent the catheter kink and improve maneuverability features. An instrument can be designed with multiple radiopaque markers placed along the catheter and/or wire as drawn by 1725.

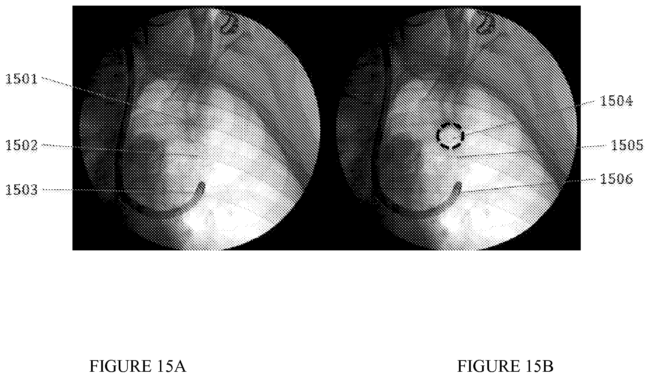

In an embodiment, **Figure 15A** shows a fluoroscopic image of the diagnostic procedure in human lungs. Biopsy needle 1502 is protruding through working channel of the bronchoscope 1503 to biopsy the suspicious target nodule, which is perceived by physician as dark region 1503. The augmented fluoroscopic image **Figure 15B** is generated to highlight the actual nodule area 1504 that was marked by physician prior to procedure on correspondent preoperative CT image of patient chest. The augmented image preserves bronchoscope 1506 and needle 1505 at the original location, however the difference between actual 1506 and perceived 1503 nodule position is obvious. The highlighting technique of 1506 is demonstrated on Figure 15B, where the yellow color is "injected" into the nodule area of the fluoroscopic image, which is correspondent to one of the CT image (and is further surrounded by a dashed line), while the original information of fluoroscopic image is yet preserved.

### Biocompatibility and Biodegradation of Foams

In some embodiments of the method described herein foams may serve as a carrier for an active pharmaceutical ingredient ("API"), e.g., as a drug and/or contrast agents. Foams including drugs and/or contrast agent should be selected so as to prevent undesired tissue reaction. For example, the foams including drugs and/or contrast agents should be compatible with mucosal tissue. The contrast agent is selected to: (1) minimize tissue reaction and/or significant inflammatory reaction and (2) have low osmolality and non-ionic characteristics, allowing for increased tissue biocompatibility. The concentration of the API used would be substantially lower than the known toxic concentration and substantially increased in view of the effective concentration of the Patient. An example of a contrast agent that may be administered orally or rectally is Barium sulphate, where Barium sulphate can be used as a foamable contrast agent. The iodine based contrast agents that may be used in the method of the present invention are non-ionic, isotonic, and can provide minimal changes in the peripheral lung parenchyma (alveoli), so as to result in significantly reduced and/or no inflammatory changes or alveolar disease. Elimination of most iodine contrast agents from the body is within the range of, e.g., 90 to 120 minutes in patients with normal renal function; however, elimination can be delayed, e.g., an the order of weeks, in patients with renal insufficiency. Elimination of iodine contrast agents can range from, e.g., 20 minutes - 120 minutes. Elimination of iodine contrast agents can range from, e.g., 20 minutes - 100 minutes. Elimination of iodine contrast agents can range from, e.g., 20 minutes - 80 minutes. Elimination of iodine contrast agents can range from, e.g., 20 minutes - 60 minutes. Elimination of iodine contrast agents can range from, e.g., 20 minutes - 40 minutes. Elimination of iodine contrast agents can range from, e.g., 40 minutes - 120 minutes. Elimination of iodine contrast agents can range from, e.g., 60 minutes - 120 minutes. Elimination of iodine contrast agents can range from, e.g., 80 minutes - 120 minutes. Elimination of iodine contrast agents can range from, e.g., 100 minutes - 120 minutes. Elimination of iodine contrast agents can range from, e.g., 40 minutes - 100 minutes. Elimination of iodine contrast agents can range from, e.g., 60 minutes - 80 minutes.

Barium sulphate is not soluble in water thus would be dispersed in the continuous phase of the foam. Iodine molecules such as iopromide and Iohexol may be readily dissolved in the water based foam. Table 1 presents commercial contrast agents that can be incorporated into the water phase in the current application of water based foamable contrast agent.

**Table 1:**

| Trade name | Active substance | Type | Organic/ Inorganic | Solubility | Osmolality (mgl/ml) |
|---|---|---|---|---|---|
| ULTRAVIST | iopromide | Iodine based monomer | Organic iodine, non-ionic | Water | 774 |
| Omnipaque | Iohexol | Iodine based monomer | Organic iodine, non-ionic | Water | 884 |
| Micropaque | Barium sulphate | Barium sulphate | TBD | Non soluble in water | TBD 100%, 1% g/ml |

Water based foams containing contrast agents provide radiogaphic contrast and avoid the biocompatibility disadvantages associated with oil carrier. The foam carrier is formulated using foaming agents selected according to their water solubility and tissue compatibility. An expandable aqueous foam formulation can coat a mucosal surface, where the expandable aqueous foam formulation includes biocompatible foaming agents (e.g., where the biocompatible foaming agents would typically not initiate tissue reaction). For example, carboxymethyl cellulose (CMC) and bovine serum albumin (BSA) are (1) compatible with mucosal tissue, (2) biocompatible and are configured to completely degrade by the body (i.e., greater than 99% degradation), and, thus, can be used as a biodegradable/bioabsorbable water-based foaming agent. Once administered, a water-based aqueous solution including CMC can sufficiently coat at least one airway, and produce minor histological changes (e.g., in comparison with poppy seed oil). Finally, the volume amount of the foaming agents in relative to the overall foam volume (e.g., which is mainly composed of gas and water) is substantially reduced. For a given diagnostic procedure that used 10 cc oil contrast agent in the Aast, the current foam contrast agent will contain >95% water and gas and <5% polymeric/ protein foaming agents.

When the foam is ejected into a body cavity, the foam begins to break into a gas phase and a liquid phase. The gas diffuses and/or evaporates, substantially reducing the volume of the foam (e.g., but not limited to, reducing the volume of the foam by 10%, 20%, 30%, 40%, 50%, 60%, 70%, 80%, 90%, 95%, etc.). The liquid phase is mainly composed (e.g., but not limited to, 60%, 70%, 80%, 90%, 95%, etc.) of water containing the foaming agent, where the foaming agent stabilizes the foam. The water is absorbed by the tissue, while the foaming agents, e.g. polymers and/or proteins, biodegrade. Therefore, the main consideration is the foaming agents and stabilizers added to the liquid phase and their concentration in the foam is selected accordingly.

### Foam and Iopromide/iodine/barium sulphate based contrast agents

Foamable contrast media was evaluated for imaging of the lungs using conventional x-ray analysis. Clear visualization of the bronchus airways was achieved using water based foam and water soluble contrast agent. The contrast agent that was tested was a non-ionic, isotonic contrast agent Omnipaque (Iohexol). Concentrations of contrast agent for obtaining substantial contrast were evaluated.

**Equipment and materials:** Initial verification of the water based foam incorporated with contrast agent concept was done using commercial Gillette Shaving foam for sensitive skin, a highly viscous foam. Omnipaque (300mg I/m1 Iohexol was used, 647mg Iohexol per lml, manufactured by GE healthcare, Ireland) was used as a contrast agent.

Two lung models were used: (1) a first model was manufactured using a rapid prototyping printer and (2) a second model was a dried Porcine Lung. Different foam/contrast agent mixed amounts were injected through a 3.5mm diameter pipe into each of the models. Standard fluoroscopic equipment was used for x-ray imaging.

**Test Procedure.** Several concentrations of Omnipaque/foam were mixed, injected through a 3.5 mm pipe. Images were recorded using a fluoroscopic camera immediately and after 10, 20, and 30 minutes post injection. Concentration of 20% Omnipaque and 80% foam measured in volume has 1/5 an its original iodine content (60mg I/m1), while 30% Omnipaque is 100mg I/m1 and 50% is 150 mg I/ml.

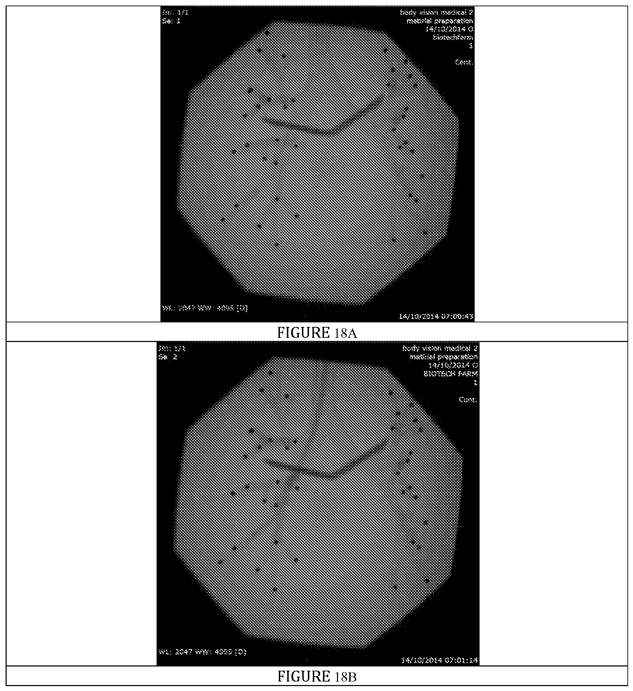

**Results:** The injection of the foamable contrast agent into the rapid prototype model was appropriate in terms of foam rheology, as the viscosity facilitate the injection pressure without interfering or breaking the foam during injection. Omnipaque is sufficiently dissolved in the water phase of the foam and produced a homogeneous solution. The foam mixture injected into the rapid prototype lung model was recorded by a fluoroscopic imaging system and is presented in Figures 18A-C.

Figure 18A: Injecting 20% Omnipaque/foam solution to a rapid prototype lung model left bronchus. Used 5cc Omnipaque to 20cc foam. No contrast was obtained.

Figure 18B: Left bronchus was injected with 30% foam/Omnipaque.

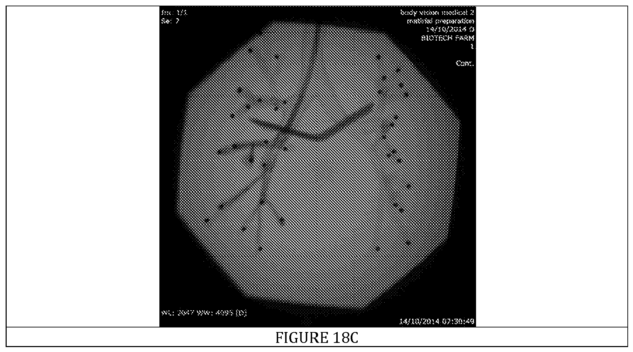

Figure 18C: Injecting 50% Omnipaque/foam solution to a rapid prototype lung model left bronchus. Used lOcc Omnipaque to lOcc foam.

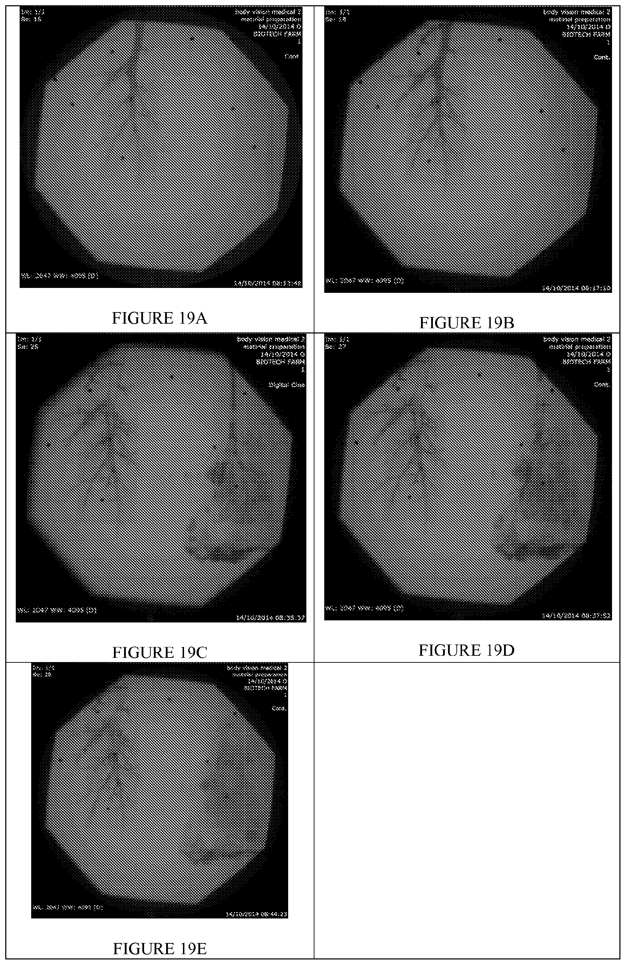

The foam contrast agent solution was sufficiently visible and clear for 10, 20, and 30 minutes post injection. The comparison between the left bronchus (i.e., foamable contrast agent) and the right bronchus (i.e., Omnipaque liquid) distinguishes the foamable contrast agent over the contrast agent in a liquid form. After a few seconds post-injection of the liquid contrast agent, the liquid sunk down due to gravity thus decreased the visibility of the lungs. The injection of the foam/contrast agent into the dry porcine lung by the fluoroscopic imaging system is presented in figures 19A-E. It is understood that the Gillette commercial foam is highly stable and other foam formulation should be selected for our application.

Figure 19A: Injecting 30% Omnipaque/foam solution to a dry porcine lung model. Image taken immediately after injection.

Figure 19B: Injecting 30% Omnipaque/foam solution to a dry porcine lung model. Image taken 4 minutes after injection.

Figure 19C: Left bronchus presents 30% Omnipaque/foam 17 minutes post injection, image was still stable. 2cc water was injected in order to wash the main bronchus. On the right bronchus liquid contrast Omnipauqe was injected and immediately recorded.

Figure 19D: Left bronchus presents 30% Omnipaque/foam 24 minutes post injection, image was still stable. The liquid Omnipauqe solution was dispersed and washed due to gravity leaving a faint image 2 minutes post its injection.

Figure 19E: Left bronchus presents 30% Omnipaque/foam about 30 minutes post injection, image was still stable. The liquid Omnipauqe solution was fully dispersed and presents a faint image, 9 minutes post its injection.

**Conclusions:** The combination between a commercial foam and contrast media allows for stable imaging over period of 30 minutes. The foam resists the gravity and is attached to the bronchus walls presenting clear images of the lungs. The 30% Omnipaque/foam solution (100 mg 1/m1) presents sufficient image quality and is appropriate in terms of rheology, injectability, resistance to gravity, stability and x-ray visibility. Omnipaque is dissolved in the water phase of the foam and produced a homogeneous solution. Other foam formulations, having lower stability than commercial shaving foam can further be tested in conjunction with the contrast agent.

### Foam-Based Contrast

Contrast agent(s) in foamable compositions is/are useful in imaging a variety of disorders of a body cavity or mucosal surfaces, including the abdominal cavity, the thoracic cavity, the cranial cavity, the ventral cavity, the vagina, penile cavities, the rectum, the urinary tract, lacrimal sacs, bladder, the cavity between the uterus and the fallopian tubes, the ovaries, uterine cavity, the ear, the nasal cavity, the mouth, the eye, peritoneum, the gastro intestinal system, salivary glands, and stomach. In an embodiment, a radiopaque contrast material is used for bronchography.

During a fluoroscopic procedure for imaging a body cavity, an image is created and captured using at least one instrument that allows perfusion of the radiopaque substance to remain visible and in place (e.g., static) for a period of time (e.g., but not limited to, 20 minutes - 120 minutes). In some embodiments, the period of time is 20 minutes - 100 minutes. In some embodiments, the period of time is 20 minutes - 80 minutes. In some embodiments, the period of time is 20 minutes - 60 minutes. In some embodiments, the period of time is 20 minutes - 40 minutes. In some embodiments, the period of time is 40 minutes - 120 minutes. In some embodiments, the period of time is 60 minutes - 120 minutes. In some embodiments, the period of time is 80 minutes - 120 minutes. In some embodiments, the period of time is 100 minutes - 120 minutes. In some embodiments, the period of time is 40 minutes - 100 minutes. In some embodiments, the period of time is 60 minutes - 80 minutes. The period of time is achieved by using at least one instrument that spreads at least one radiopaque substance an the walls of airways using a pressurized aerosol foamable contrast agent. The radiopaque material is dissolved or dispersed in the aqueous phase of water-based foam. The radiopaque substance is homogenously dispersed in the foam, so as to produce sufficient contrast in a body cavity. The radiopaque substance having a relatively high viscosity is ejected through a canister-pipe mechanism and is dispersed onto the area of interest of the lung. The airway area retains a radiopaque characteristic for an increased period of time compared to typically used contrast agents. The foam-based radiopaque material is able to provide sufficient contrast describing the contour of the body cavity for example in lung bronchus. The foam remains stable for an increased period of time (e.g., but not limited to, about 20 - 120 minutes) while preventing fluoroscopic image quality deterioration compared to typical liquid based contrast injection. The foam based contrast agent is not substantially influenced by gravity due to its high surface tension that holds the foam, and thereby the contrast agent remains stable in place until the foam breaks. The foam stability determines the duration in which stable contrast is obtained. In addition, the viscosity of the foam is formulated so as to facilitate effective injection of the foam into the bronchus.

A foamable contrast agent was prepared using 30% v/v water soluble contrast agent (Omnipaque, 300mg 1/m1 Iohexol) incorporated in the aqueous phase thus the iodine content of the foam is 60 mg I/ml. Foaming agents that were used are carboxy methyl cellulose (CMC), bovine serum albumin (BSA), and Agar. The foam remained stable for 10 and 20 minutes using different formulations. The concentrations used are 0.02-0.05% (w/v) BSA with the mixed polymer 0.6-0.1% CMC and 0.2-0.5% agar added to the aqueous solution. In some instances the solution was heated to 40°C and stirred so as to obtain homogenous solution. In one instance the foam was stirred using a home mixing tools for about 10 minutes, while in another instance foam was produced using a home cream whipper (KAYSER, Austria) and a Nitrous oxide (N20) charger. With the latter, the mixture was poured into a closed canister and N20 was injected into the canister. Other water soluble contrast agents that were used are iopromide and Barium sulphate. Both were added within the water phase. Barium sulphate produced a white, opaque solution and was not dissolved in the water phase.

### Decomposition of Foams

In an embodiment of the method described herein a radiopaque contrast material is dissolved in a water-based foam, where the water-based foam is configured to remain stable during predefined period of time following the injection inside the area of interest of natural body cavity. The physical stability of the foam fluid contrast medium is maintained over the course of the imaging, thus stabilities of both individual bubbles and the bubble dispersion are taken into consideration. The stability time of the foam fluid is the time duration during which the bubble density differences would not cause fluoroscopic image quality deterioration or blurred fluoroscopic image. The foam is required to be stable for 30 to 90 minutes for clinical applications.

Fluid foam is a dispersion of large amount of gas bubbles distributed in a liquid phase. Since two foam phases are not miscible, they are not thermodynamically stable and hence are typically stabilized using a surface-active agent. Upon valve actuation from a closed gas pressurized foam, the foam is ejected and mixed with air, and there is a generation of air bubbles that produce new air/water interfaces, and hence free energy increases. By controlling its bubble structure, one can obtain desired rheological properties such as viscosity and injectability.

In addition, surface-active agent reduces the surface free energy generated when a foam is formed and increases foam stability. The stability of foams is a complex interplay between several mechanisms such as Ostwald ripening, drainage, film rupture or bubble coalescence, and bubble flow. Drainage is the irreversible flow of liquid through the foam in the direction of the gravity vector. The net result of this process is that the average bubble size grows in time. When the film between two bubbles ruptures, the bubble size is increased eventually the foam will collapse and vanish.

Foamability is the ability of a solution to produce foam, while foam stability refers to lifetime, or duration that the foam retains its structure post its generation. The presence of a foaming agent is essential for foam generation and stabilization. Foaming agents are amphiphilic substances while the hydrophilic part of a molecule is responsible for their solubility in water and their hydrophobic part of the molecule arrange itself so to stabilize the foam structure by reducing surface are around the bubbles, and thus reducing surface tension between the gas/ liquid interface. When a foaming agent is adsorbed into the gas/water interface, the surface tension of water is lowered and the gas bubbles which are the dispersed phase may preserve their size and structure for longer duration.

Foaming agents are surface active agents that produce steric and or electrostatic stabilization for the film that is generated between gas bubbles. Frequently one active agent is not enough for foam stability, thus it is necessary to add more than one component to increase foam lifetime. Addition of polymers to surfactant solutions can also significantly enhance the foam stability owing to complex formation of the components. Surface active polymers increase the surface viscosity due to complex formation between the surface active agents and polymer at the liquid/gas surface and thereby increase the film stability. The addition of polymer typically increases the bulk viscosity, which may also increase the drainage time of the film and enhanced long-term stability. Proteins are suitable foaming agents since they strongly adsorb to the gas-water interface, they tend produce and the adsorbed film increases its structural coherence (high surface rheological moduli). Proteins adsorb at the air-water interface because they possess hydrophobic regions due to the hydrophobic amino acid residues. Proteins unfolding due to heat, addition of surface active agents may further increase the stability of the foam. For example, bovine serum albumin (BSA) has been successfully used as a drug carrier and stabilization agent for microbubbles based stabilization agents.

Specifically, the following foaming agents may be used in order to obtain longer stability and thereby longer fluoroscopic imaging: anionic-sodium dodecyl sulphate (SDS), Gelatin, Agar, polyvinyl alcohol, polyvinyl pyridine, sodium carboxyl methyl cellulose, Tween 80, Polysorbate 80, and Xanthan gum, a polysaccharide secreted by the bacterium Xanthomonas. Gluconolactone may be added so as to increase stability and therefore increase the drainage duration of albumin or CMC containing foams.

In some embodiments, the instant invention is a method and flow that allows using first imaging modality such as CT, MRI, etc., and planning information through generation of augmented image from second imaging modality, such as fluoroscopy, digital subtraction angiography (DSA), etc., with highlighted area of interest or structures and optionally additional imaging and\or planning information, originated from a first imaging modality, superimposed over it comprising: (i) using first imaging modality to obtain at least one first image of chest; (ii) manual or automatic planning of procedure through defining landmarks, area of interest, incision points, critical structures, bifurcations, anatomical organs, etc.; (iii) acquire at least one-second image from second imaging modality, such as fluoroscopy or DSA, and generation of compatible virtual image, such as DRR, from first imaging modality; (iv) mapping of planning data to the objects and structures on the compatible virtual image; (v) registration of at least one second image or video frame from second imaging modality to first image or its portion sourced from first imaging modality; (vi) transfer mapping (i.e., identifying and mapping) of planning data from the compatible virtual image, sourced from first imaging modality to second image from second imaging modality by means of image registration; (vii) highlighting the area of interest, anatomical structures on second image sourced from second imaging modality to obtain third image, wherein the third image is augmented.

In some embodiments, the method further includes superimposing of at least one image or its derivative, it's portion or image based planning information sourced from first imaging modality over second imaging modality. In some embodiments, the method further includes navigation and guidance instructions that aid movement of medical instrument. In some embodiments, the method further includes guidance for positioning second imaging modality, such as fluoroscopic C-Arm, to allow maintaining optimal visibility for the area of interest, incision points, anatomical structures, tool access direction. In some embodiments, the method implements tracking of anatomic structures on subsequent frames from second imaging modality, such as fluoroscopic video, having same acquisition parameters (mode, position, field of view) to allow higher quality of augmented fluoroscopic image through suppression of static anatomic structures and improving signal to noise of underlying soft tissue. In some embodiments, multiphase registration is performed, where the static objects with small movement, such as ribs, are registered at first and more dynamic objects such as diaphragm, bronchi, blood vessels, etc. are gradually registered in the following registration iterations. In some embodiments, the interfering structures being deemphasized. In some embodiments, the compatible virtual image is not generated while the planning data from first imaging modality is transferred to second imaging modality by means of image registration.

In some embodiments, a method is described allowing for the generation of an augmented third image, such as intraoperative fluoroscopic, DSA, etc., with highlighted area of interest or structures comprising: (i) using at least two intraoperative images with known relative movement and rotation to allow grouping pixels of intraoperative image according to their movement variation and intensity values; (ii) performing registration or cross-correlation between at least two sequential intraoperative images to reconstruct structures in the area of interest; (iii) differentiating moving and static structures in the area of interest on user demand; (iv) highlighting anatomical structures on intraoperative image, or any combination thereof. In some embodiments, the method includes using Chest X-ray radiographic image, while the said radiographic image serves as a reference image that enables to enhance anatomical structures on second image through registration or cross-correlation of the information from radiographic image.

In some embodiments, an augmented fluoroscopy device is described that allows generation of augmented fluoroscopy image comprising: a video and image processing unit; a video input card or externally connected device that is capable to input video signal from the variety of Fluoroscopic device; a 3D planning input in internal or DICOM format; an augmented video signal output; or any combination thereof.

In some embodiments, the device is integrated within fluoroscopic device as a module, to obtain RAW data as a signal, and therefore having RAW data input card instead of video input card. In some embodiments, the device is integrated within cone-beam CT system.

In some embodiments, the present invention may be used as a tissue or anatomical structure highlighting technique, where the volume of interest is selected on the image sourcing from first imaging modality, such as CT or MRI; acquired image from second imaging modality; coarse registration is performed between second and first imaging modalities to identify the pose of virtual camera in the second imaging modality correspondent to the one of second imaging modality; at least one pattern is produced from first imaging modality for the anatomical structure around volume of interest; the matching pattern is found in the second imaging modality using single or multiple patterns produced from first imaging modality; the matching pattern from the second imaging modality is enhanced to highlight the anatomy in the volume of interest producing third imaging modality.

In some embodiments of the method described herein, when the anatomic structures located outside the area of interest are found and suppressed using the same technique. In some embodiments, the pattern is comprised from anatomical features such as airways, ribs, and blood vessels. In some embodiments, the matching feature from second imaging modality is derived from set of at least one instrument position inside the area of interest.

A method of object depth calculation as follows: given the Parameters of compatible virtual image sourcing from first imaging modality, such as DRR - to fluoroscopy; given the pose and field of view of virtual camera, such as virtual fluoroscopic camera, projecting first imaging modality to second imaging modality; determine the object size on virtual image, such as ribs width on DRR at specific location; calculate the depth (such as distance of the specific object or object area from fluoroscopic X-ray source) through comparison between the known object sizes sourced from first image (e.g. CT image) to the one measured on second image (e.g. fluoroscopic image), or any combination thereof. In some embodiments, object size is determined from technical specification instead of or in addition to the measurement on compatible virtual image, such as tool rigid part length or width. In some embodiments, the catheter-type tool is designed to allow the calculation of trajectory as a combination of depth distances from second imaging modality camera center.

A method and flow that allow registration of first three-dimensional imaging modality such as CT, MRI, etc., with second two-dimensional imaging modality of real time x-ray imaging such as fluoroscopy, digital subtraction angiography (DSA), etc. comprising: using first imaging modality to obtain at least one first image of chest; perform manual or automatic segmentation of natural body cavities such as bronchial airways in 3D space; acquire at least one images or sequence of video frames from second imaging modality, such as fluoroscopy or DSA; generation of two-dimensional augmented image generated from second imaging modality that combines unique information to describe the full or partial map of natural body cavities such as portion of bronchial airway tree, abovementioned as augmented bronchogram; calculate registration between first and second imaging modalities through pose estimation by fitting abovementioned corresponded features, or any combination thereof. In some embodiments, an augmented bronchogram is generated using radiopaque material is injected to highlight the body cavity.

In some embodiments, augmented bronchogram is generated through superposition of imaging from at least two different temporal positions of radiopaque instrument located inside the body cavity. In some embodiments, augmented bronchogram is generated through superposition of imaging from at least one different positions of radiopaque instrument located inside the body cavity and angular measurement of C-Arm orientation relative to patient bed. In some embodiments, the radiopaque instrument is designed and configured to reconstruct its three-dimensional space from single projection. In some embodiments, radiopaque substances having a high viscosity such as, but not limited to, hydrogel, reverse thermo-gelling polymer are used to generate augmented bronchogram. In some embodiments, superposition of imaging incorporates distortion correction caused by body movement, breathing, instrument introduction etc. In some embodiments, the temporal instrument positions are acquired for superposition at the predefined breathing phase. In some embodiments, the present invention is a device for navigating inside natural body cavity comprising: guided sheath with anchoring at the tip and guided wire. In some embodiments, the device includes an inflatable balloon serving as anchoring mechanism.

In some embodiments, a method is described, the method including:
obtaining a first image from a first imaging modality; identifying on the first image from the first imaging modality at least one element, where the at least one element comprises a landmark, an area of interest, an incision point, a bifurcation, an organ, or any combination thereof, obtaining a second image from a second imaging modality; generating a compatible virtual image from the first image from the first imaging modality; mapping planning data on the compatible virtual image; where mapped planning data corresponds to the at least one element, coarse registering of the second image from the second imaging modality to the first image from the first imaging modality; identifying at least one element of the mapped planning data from the compatible virtual image; identifying at least one corresponding element on the second imaging modality; mapping the at least one corresponding element on the second imaging modality; fine registering of the second image from the second imaging modality to the first image from the first imaging modality; generating a third image; where the third image is an augmented image including a highlighted area of interest.

In some embodiments, the method further includes superimposing the at least one image, a portion of the at least one image, or a planning information derived from the first imaging modality over the second imaging modality. In some embodiments, the method further includes using at least one instruction, where the at least one instruction can include information regarding navigation, guidance, or a combination thereof. In some embodiments, the guidance includes information regarding a positioning of a device shown the second imaging modality, where the device comprises a fluoroscopic C-Arm, as to result in achieving visibility for the area of interest, incision points, anatomical structures, or tool access direction. In some embodiments, the method further includes tracking of at least one anatomical structure by use of at least one subsequent image derived from the second imaging modality, where the second imaging modality comprises a fluoroscopic video configured to have substantially the same acquisition parameters, and where the acquisition parameters comprise mode, position, field of view, or any combination thereof, to generate the augmented fluoroscopic image by suppressing static anatomic structures and/or improving signal to noise of underlying soft tissue. In some embodiments, the method further includes performing a multiphase registration, where the at least one substantially static object is first registered; and where at least one dynamic object is second registered, where the at least one dynamic object comprises a diaphragm, a bronchus, a blood vessel, or any combination thereof. In some embodiments, the method further includes deemphasizing at least one interfering structure. In some embodiments, the compatible virtual image is not generated while the planning data from first imaging modality is transferred to second imaging modality by means of image registration.

In some embodiments, a method is described, the method including:
using at least two intraoperative images with known relative movement and rotation to generate a grouping of pixels derived from an intraoperative image, where the grouping of pixels is determined by individual calculation of each pixel using: (a) movement variation of each pixel and (b) intensity values of each pixel; performing registration using at least two sequential intraoperative images to reconstruct structures in an area of interest; differentiating moving structures from static structures in the area of interest; and highlighting anatomical structures an at least one intraoperative image. In some embodiments, the method further includes using a chest x-ray radiographic image as a first intraoperative image.

In some embodiments, the instant invention may be used in conjunction with a system including an augmented fluoroscopy device configured to generate an augmented fluoroscopy image including (a) video and image processing unit, (b) video input card or externally connected device configured to input video signal a fluoroscopic device, (c) 3D planning input in internal or DICOM format, (d) an augmented video signal output, or any combination thereof. In some embodiments, the system is integrated with at least one fluoroscopic device is a module including a RAW data input card (i.e., instead of a video input card) configured to obtain RAW data as a signal. In some embodiments, the system is integrated with a Cone-beam CT system.

In some embodiments, the instant invention may be used in conjunction with a system including an instrument for navigating inside natural body cavity including: (a) a guided sheath with anchoring at the tip and/or (b) a guided wire. In some embodiments, the instrument is an inflatable balloon configured to act as an anchoring mechanism.

In some embodiments, a method is provided, the method including: (i) selecting a volume of interest an a first image from a first imaging modality; (ii) generating a second image from a second imaging modality; (iii) coarse registering using the first imaging modality and the second imaging modality; (iv) producing at least one pattern from the first imaging modality; (v) generating a matching pattern by use of the second imaging modality using single or multiple patterns produced from first imaging modality; (vi) enhancing the matching pattern from the second imaging modality to highlight the anatomy in the volume of interest for producing third imaging modality. In some embodiments, the anatomic structures located outside the area of interest are found and suppressed using substantially the same method. In some embodiments, the pattern includes anatomical features including, but not limited to, airways, ribs, and blood vessels. In some embodiments, the matching feature from second imaging modality is derived from a set of at least one instrument position inside the area of interest.

In some embodiments, a method is provided, the method including: using a first imaging modality to obtain at least one first image of a patient's chest; segmenting natural body cavities including bronchial airways in a 3D space; generating at least one image from a second imaging modality; generating a two-dimensional augmented image generated from the second imaging modality by combining information, where the information describes a complete map or a partial map of natural body cavities, including a bronchial airway tree; calculating registration between the first imaging modality and the second imaging modality as pose estimation between the Portion of bronchial airway sourcing from second imaging modality and segmented map of bronchial airway sourcing from first imaging modality; calculating registration between first and second imaging modalities through pose estimation by mapping corresponding features. In some embodiments, the augmented bronchogram is generated using radiopaque material is injected to highlight the body cavity. In some embodiments, the augmented bronchogram is generated through superposition of imaging from at least three two different positions of radiopaque instrument located inside the body cavities. In some embodiments, an augmented bronchogram is generated through superposition of imaging from at least one different positions of radiopaque instrument located inside the body cavity and angular measurement of C-Arm orientation relative to patient bed. In some embodiments, the radiopaque instrument is designed and configured to reconstruct its three-dimensional space from single projection. In some embodiments, the radiopaque substance(s) having a high viscosity such as, but not limited to, hydrogel, reverse thermo-gelling polymer can be used to generate augmented bronchogram.

In some embodiments, a method is provided, the method including:
providing the Parameters of compatible virtual image sourcing from first imaging modality, such as, but not limited to, DRR - to fluoroscopy; determining an object size on virtual image, such as, but not limited to, ribs width on DRR at specific location; providing the pose and field of view of a virtual camera, such as, but not limited to, a virtual fluoroscopic camera, projecting first imaging modality to second imaging modality such as fluoroscopic camera calculated from calibration process; determining the object size on the virtual image, such as ribs width on DRR at specific location; calculating the depth (for example, but not limited to, distance of the specific object or object area from fluoroscopic X-ray source) through comparison between the known object sizes sourced from first image (e.g. CT image) to the one measured on second image (e.g. fluoroscopic image). In some embodiments, the object size is determined from technical specification instead of or in addition to the measurement on compatible virtual image, such as tool rigid part length or width. In some embodiments, the catheter-type tool is designed to allow the calculation of trajectory as a combination of depth distances from second imaging modality camera center.

In some embodiments, a method is provided, the method including:
introducing a foam compound into a cavity of a subject; obtaining a first image from a first imaging modality; where the first image from the first imaging modality includes a first radiopaque image derived from the foam compound, identifying on the first image from the first imaging modality at least one element, where the at least one element includes a landmark, an area of interest, an incision point, a bifurcation, an organ, or any combination thereof, obtaining a second image from a second imaging modality; where second first image from the second imaging modality includes a second radiopaque image derived from the foam compound, generating a compatible virtual image from the first image from the first imaging modality; mapping planning data on the compatible virtual image; where mapped planning data corresponds to the at least one element, coarse registering of the second image from the second imaging modality to the first image from the first imaging modality; identifying at least one element of the mapped planning data from the compatible virtual image; identifying at least one corresponding element on the second imaging modality; mapping the at least one corresponding element on the second imaging modality; fine registering of the second image from the second imaging modality to the first image from the first imaging modality; generating a third image; where the third image is an augmented image including a highlighted area of interest. In some embodiments, the method further includes superimposing the at least one image, a portion of the at least one image, or a planning information derived from the first imaging modality over the second imaging modality. In some embodiments, the method further includes using at least one instruction, where the at least one instruction can include information regarding navigation, guidance, or a combination thereof. In some embodiments, the guidance includes information regarding a positioning of a device shown the second imaging modality, where the device includes a fluoroscopic C-Arm, as to result in achieving visibility for the area of interest, incision points, anatomical structures, or tool access direction. In some embodiments, the method further includes tracking of at least one anatomical structure by use of at least one subsequent image derived from the second imaging modality, where the second imaging modality includes a fluoroscopic video configured to have substantially the same acquisition parameters, and where the acquisition parameters include mode, position, field of view, or any combination thereof, to generate the augmented fluoroscopic image by suppressing static anatomic structures and/or improving signal to noise of underlying soft tissue. In some embodiments, the method further includes performing a multiphase registration, where the at least one substantially static object is first registered; and where at least one dynamic object is second registered, where the at least one dynamic object includes a diaphragm, a bronchus, a blood vessel, or any combination thereof. In some embodiments, the method further includes deemphasizing at least one interfering structure. In some embodiments, the compatible virtual image is not generated while the planning data from first imaging modality is transferred to second imaging modality by means of image registration.

In some embodiments, a method is provided, the method including:
introducing an instrument into a cavity of a subject; obtaining a first image from a first imaging modality; where the first image from the first imaging modality includes a first radiopaque image derived from the instrument, identifying an the first image from the first imaging modality at least one element, where the at least one element includes a landmark, an area of interest, an incision point, a bifurcation, an organ, or any combination thereof, obtaining a second image from a second imaging modality; where second first image from the second imaging modality includes a second radiopaque image derived from the instrument, generating a compatible virtual image from the first image from the first imaging modality; mapping planning data on the compatible virtual image; where mapped planning data corresponds to the at least one element, coarse registering of the second image from the second imaging modality to the first image from the first imaging modality; identifying at least one element of the mapped planning data from the compatible virtual image; identifying at least one corresponding element on the second imaging modality; mapping the at least one corresponding element on the second imaging modality; fine registering of the second image from the second imaging modality to the first image from the first imaging modality; generating a third image; where the third image is an augmented image including a highlighted area of interest. In some embodiments, the method further includes superimposing the at least one image, a portion of the at least one image, or a planning information derived from the first imaging modality over the second imaging modality. In some embodiments, the method further includes using at least one instruction, where the at least one instruction can include information regarding navigation, guidance, or a combination thereof. In some embodiments, the guidance includes information regarding a positioning of a device shown the second imaging modality, where the device includes a fluoroscopic C-Arm, as to result in achieving visibility for the area of interest, incision points, anatomical structures, or tool access direction. In some embodiments, the method further includes tracking of at least one anatomical structure by use of at least one subsequent image derived from the second imaging modality, where the second imaging modality includes a fluoroscopic video configured to have substantially the same acquisition parameters, and where the acquisition parameters include mode, position, field of view, or any combination thereof, to generate the augmented fluoroscopic image by suppressing static anatomic structures and/or improving signal to noise of underlying soft tissue. In some embodiments, the method further includes performing a multiphase registration, where the at least one substantially static object is first registered; and where at least one dynamic object is second registered, where the at least one dynamic object includes a diaphragm, a bronchus, a blood vessel, or any combination thereof. In some embodiments, the method further includes deemphasizing at least one interfering structure. In some embodiments, the compatible virtual image is not generated while the planning data from first imaging modality is transferred to second imaging modality by means of image registration.

While a number of embodiments of the present invention have been described, it is understood that these embodiments are illustrative only, and not restrictive, and that many modifications may become apparent to those of ordinary skill in the art. Further still, the various steps may be carried out in any desired order (and any desired steps may be added and/or any desired steps may be eliminated).

## Claims

1. A system for generating an augmented fluoroscopy image, said system comprising:
- means for introducing a foam compound into a cavity of a subject; wherein the foam compound comprises a radiopaque substance and is configured to be attached to a wall of the cavity of the subject describing a contour of the cavity of the subject,
- means for obtaining a first image from a first imaging modality;
wherein the first image from the first imaging modality is a first radiopaque image generated from the foam compound,
wherein the first radiopaque image generated from the foam compound is a three-dimensional preoperative computer tomographic image,
- means for identifying on the first image from the first imaging modality at least one element, wherein the at least one element corresponds to a unit of anatomy that has a common mechanical characteristic, wherein the at least one element comprises an area of interest,
- means for obtaining a second image from a second imaging modality;
wherein the second image from the second imaging modality comprises a second radiopaque image generated from the foam compound,
wherein the second radiopaque image generated from the foam compound is a two-dimensional intraoperative fluoroscopic image,
- means for generating a compatible virtual image from the first image from the first imaging modality;
wherein the compatible virtual image is a compatible digital reconstructed radiograph image being generated from the first image using a pose and field of view of a virtual camera in the second imaging modality in order to obtain information of the at least one element identified on the first image regarding size or depth,
- means for mapping planning data on the compatible virtual image;
wherein mapping planning data on the compatible virtual image corresponds to transferring planning data on the compatible virtual image,
wherein planning data is pre-procedure data corresponding to a surgical treatment or diagnosis,
wherein mapped planning data corresponds to the at least one element identified on the first image,
- means for coarse registering of the second image from the second imaging modality to the first image from the first imaging modality; wherein coarse registering corresponds to a rough alignment of the first image from the first imaging modality and the second image from the second imaging modality using global information in order to identify the pose of the virtual camera in the second imaging modality,
- means for identifying the at least one element of the mapped planning data from the compatible virtual image, the at least one element of the mapped planning data corresponding to the at least one element identified on the first image;
- means for identifying at least one corresponding element on the second imaging modality;
wherein at least one corresponding element means at least one element corresponding to the at least one element of the first image and the virtual image,
- means for mapping the at least one corresponding element on the second imaging modality;
- means for fine registering of the second image from the second imaging modality to the first image from the first imaging modality; wherein fine registering refers to a registration of a local tissue around the area of interest of the first image, which corresponds to an area of the second image,
- means for generating a third image;
wherein the third image is the augmented fluoroscopic image,
wherein the third image corresponds to the second image from the second imaging modality including a highlighted area of interest,
wherein the third image is obtained by adding at least one three-dimensional aspect of information to the second image,
wherein the at least one three-dimensional aspect of information corresponds to the anatomical and/or the planning data extracted from the first image and/or the compatible virtual image,
- a monitor configured to display the third image.

2. The system of claim 1,
further comprising means for performing a multiphase registration,
wherein at least one substantially static object is first registered; and
wherein at least one dynamic object is second registered, wherein the at least one dynamic object comprises a diaphragm, a bronchus, a blood vessel, or any combination thereof, and wherein the static object comprises ribs.

3. The system of claim 1 or 2,
further comprising means for deemphasizing at least one interfering structure on the third image, wherein the at least one interfering structure is any structure that could interfere with an anatomical focus of a procedure.

4. The system of claim 1 or 2, further comprising:
- an X-ray tube for generating X-ray beams;
- a collimator that is designed to narrow the X-ray beams generated by the X-ray tube;
- an X-ray image intensifier for absorbing attenuated X-ray beams; and
- a C-arm for movement of frame with attached fluoroscopic pair of X-ray tube and intensifier.

5. The system of claim 4,
further comprising means for converting a raw data fluoroscopic image into a visible image, wherein the raw data fluoroscopic image is formed by the X-ray image intensifier and the visible image is the second image that is the intraoperative fluoroscopic image.

6. The system of claim 4 or 5,
further comprising a planning station for planning the diagnostic and/or treatment procedure on the basis of the first image that is the preoperative computer tomographic image, and for generating planning data.

## Patentansprüche

1. System zur Erzeugung eines erweiterten Durchleuchtungsbildes, wobei das System Folgendes aufweist:
- Mittel zum Einführen einer Schaumverbindung in eine Kavität eines Subjekts;
wobei die Schaumverbindung eine Kontrastsubstanz aufweist und zum Anbringen an einer Wand der Kavität des Subjekts konfiguriert ist, wobei sie eine Kontur der Kavität des Subjekts beschreibt,
- Mittel zur Gewinnung eines ersten Bildes von einer ersten Bildgebungsmodalität;
wobei das erste Bild von der ersten Bildgebungsmodalität ein erstes strahlungsundurchlässiges Bild ist, das von der Schaumverbindung erzeugt wird,
wobei das erste strahlungsundurchlässige Bild, das von der Schaumstoffverbindung erzeugt wird, ein dreidimensionales präoperatives computertomographisches Bild ist,
- Mittel, um auf dem ersten Bild von der ersten Bildgebungsmodalität mindestens ein Element zu identifizieren, wobei das mindestens eine Element einer Einheit der Anatomie entspricht, die eine gemeinsame mechanische Eigenschaft aufweist, wobei das mindestens eine Element einen Bereich von Interesse aufweist,
- Mittel zur Gewinnung eines zweiten Bildes von einer zweiten Bildgebungsmodalität;
wobei das zweite Bild von der zweiten Bildgebungsmodalität ein zweites strahlungsundurchlässiges Bild aufweist, das von der Schaumverbindung erzeugt wird,
wobei das zweite strahlungsundurchlässige Bild, das von der Schaumverbindung erzeugt wird, ein zweidimensionales intraoperatives Durchleuchtungsbild ist,
- Mittel zum Erzeugen eines kompatiblen virtuellen Bildes aus dem ersten Bild von der ersten Bildgebungsmodalität;
wobei das kompatible virtuelle Bild ein kompatibles digitales rekonstruiertes Röntgenbild ist, das aus dem ersten Bild unter Verwendung einer Position und eines Sichtfeldes einer virtuellen Kamera in der zweiten Bildgebungsmodalität erzeugt wird, um Informationen über das mindestens eine auf dem ersten Bild identifizierte Element hinsichtlich Größe oder Tiefe zu erhalten,
- Mittel zum Abbilden von Planungsdaten auf dem kompatiblen virtuellen Bild;
wobei das Abbilden von Planungsdaten auf dem kompatiblen virtuellen Bild dem Übertragen von Planungsdaten auf das kompatible virtuelle Bild entspricht,
wobei die Planungsdaten Daten vor dem Eingriff entsprechend einer chirurgischen Behandlung oder Diagnose sind,
wobei die abgebildeten Planungsdaten dem mindestens einen auf dem ersten Bild identifizierten Element entsprechen,
- Mittel zur Grobregistrierung des zweiten Bildes von der zweiten Bildgebungsmodalität auf dem ersten Bild von der ersten Bildgebungsmodalität;
wobei die Grobregistrierung einer groben Ausrichtung des ersten Bildes von der ersten Bildgebungsmodalität und des zweiten Bildes von der zweiten Bildgebungsmodalität unter Verwendung globaler Informationen entspricht, um die Position der virtuellen Kamera in der zweiten Bildgebungsmodalität zu identifizieren,
- Mittel zum Identifizieren des mindestens einen Elements der abgebildeten Planungsdaten von dem kompatiblen virtuellen Bild, wobei das mindestens eine Element der abgebildeten Planungsdaten dem mindestens einen auf dem ersten Bild identifizierten Element entspricht;
- Mittel zum Identifizieren mindestens eines entsprechenden Elements auf der zweiten Bildgebungsmodalität;
wobei mindestens ein entsprechendes Element mindestens ein Element bedeutet, das dem mindestens einen Element des ersten Bildes und des virtuellen Bildes entspricht,
- Mittel zum Abbilden des mindestens einen entsprechenden Elements auf der zweiten Bildgebungsmodalität;
- Mittel zur Feinregistrierung des zweiten Bildes von der zweiten Bildgebungsmodalität auf dem ersten Bild von der ersten Bildgebungsmodalität;
wobei sich die Feinregistrierung auf eine Registrierung eines lokalen Gewebes um den Bereich von Interesse des ersten Bildes bezieht, der einem Bereich des zweiten Bildes entspricht,
- Mittel zum Erzeugen eines dritten Bildes;
wobei das dritte Bild das erweiterte Durchleuchtungsbild ist,
wobei das dritte Bild dem zweiten Bild der zweiten Bildgebungsmodalität entspricht, das einen hervorgehobenen Bereich von Interesse aufweist,
wobei das dritte Bild durch Hinzufügen mindestens eines dreidimensionalen Informationsaspekts zu dem zweiten Bild erhalten wird,
wobei der mindestens eine dreidimensionale Informationsaspekt den anatomischen und/oder den Planungsdaten entspricht, die aus dem ersten Bild und/oder dem kompatiblen virtuellen Bild extrahiert wurden,
- einen Monitor, der zur Anzeige des dritten Bildes konfiguriert ist.

2. System nach Anspruch 1,
ferner aufweisend Mittel zum Durchführen einer mehrphasigen Registrierung, wobei mindestens ein im Wesentlichen statisches Objekt zuerst registriert wird; und wobei mindestens ein dynamisches Objekt als zweites registriert wird, wobei das mindestens eine dynamische Objekt ein Zwerchfell, eine Bronchie, ein Blutgefäß oder eine beliebige Kombination davon aufweist, und wobei das statische Objekt Rippen aufweist.

3. System nach Anspruch 1 oder 2,
das ferner Mittel aufweist um mindestens einer störenden Struktur auf dem dritten Bild weniger Bedeutung beizumessen, wobei die mindestens eine störende Struktur eine beliebige Struktur ist, die einen anatomischen Fokus eines Verfahrens stören könnte.

4. System nach Anspruch 1 oder 2, welches ferner Folgenes aufweist:
- eine Röntgenröhre zur Erzeugung von Röntgenstrahlen;
- einen Kollimator, der so ausgelegt ist, dass er die von der Röntgenröhre erzeugten Röntgenstrahlen verengt;
- einen Röntgenbildverstärker zum Absorbieren der abgeschwächten Röntgenstrahlen; und
- einen C-Bogen zur Bewegung des Rahmens mit dem daran befestigten Durchleuchtungspaar aus Röntgenröhre und Verstärker.

5. System nach Anspruch 4,
das ferner Mittel zum Umwandeln eines Rohdaten-Durchleuchtungsbildes in ein sichtbares Bild aufweist, wobei das Rohdaten-Durchleuchtungsbild durch den Röntgenbildverstärker erzeugt wird und das sichtbare Bild das zweite Bild ist, das das intraoperative Durchleuchtungsbild ist.

6. System nach Anspruch 4 oder 5,
ferner mit einer Planungsstation zur Planung des Diagnose- und/oder Behandlungsverfahrens auf der Grundlage des ersten Bildes, das das präoperative computertomographische Bild ist, und zur Erzeugung von Planungsdaten.

## Revendications

1. Système de génération d'une image fluoroscopique augmentée, ledit système comprenant :
- un moyen d'introduction d'un composé de mousse jusque dans une cavité d'un sujet ;
dans lequel le composé de mousse comprend une substance radio-opaque et est configuré pour être attaché à une paroi de la cavité du sujet décrivant un contour de la cavité du sujet,
- un moyen d'obtention d'une première image provenant d'une première modalité d'imagerie ;
dans lequel la première image provenant de la première modalité d'imagerie est une première image radio-opaque générée à partir du composé de mousse,
dans lequel la première image radio-opaque générée à partir du composé de mousse est une image préopératoire tridimensionnelle de tomographie assistée par ordinateur,
- un moyen d'identification, sur la première image provenant de la première modalité d'imagerie, d'au moins un élément, dans lequel ledit au moins un élément correspond à une unité d'anatomie qui a une caractéristique mécanique commune,
dans lequel ledit au moins un élément comprend une zone d'intérêt,
- un moyen d'obtention d'une deuxième image à partir d'une deuxième modalité d'imagerie ;
dans lequel la deuxième image provenant de la deuxième modalité d'imagerie comprend une deuxième image radio-opaque générée à partir du composé de mousse,
dans lequel la deuxième image radio-opaque générée à partir du composé de mousse est une image fluoroscopique peropératoire bidimensionnelle,
- un moyen de génération d'une image virtuelle compatible à partir de la première image provenant de la première modalité d'imagerie ;
dans lequel l'image virtuelle compatible est une image radiographique numérique reconstruite compatible ayant été générée à partir de la première image en utilisant une pose et un champ de vision d'une caméra virtuelle dans la deuxième modalité d'imagerie afin d'obtenir des informations dudit au moins un élément identifié sur la première image concernant une taille ou une profondeur,
- un moyen de mappage de données de planification sur l'image virtuelle compatible ;
dans lequel le mappage de données de planification sur l'image virtuelle compatible correspond à un transfert de données de planification sur l'image virtuelle compatible,
dans lequel les données de planification sont des données de pré-procédure correspondant à un traitement ou diagnostic chirurgical, dans lequel les données de planification mappées correspondent audit au moins un élément identifié sur la première image,
- un moyen d'enregistrement grossier de la deuxième image provenant de la deuxième modalité d'imagerie vers la première image provenant de la première modalité d'imagerie ;
dans lequel l'enregistrement grossier correspond un alignement approximatif de la première image provenant de la première modalité d'imagerie et de la deuxième image provenant de la deuxième modalité d'imagerie en utilisant des informations globales afin d'identifier la pose de la caméra virtuelle dans la deuxième modalité d'imagerie,
- un moyen d'identification dudit au moins un élément des données de planification mappées à partir de l'image virtuelle compatible, ledit au moins un élément des données de planification mappées correspondant audit au moins un élément identifié sur la première image ;
- un moyen d'identification d'au moins un élément correspondant sur la deuxième modalité d'imagerie ;
dans lequel au moins un élément correspondant signifie au moins un élément correspondant audit au moins un élément de la première image et de l'image virtuelle,
- un élément de mappage dudit au moins un élément correspondant sur la deuxième modalité d'imagerie ;
- un élément d'enregistrement précis de la deuxième image provenant de la deuxième modalité d'imagerie vers la première image provenant de la première modalité d'imagerie ;
dans lequel l'enregistrement précis se réfère à un enregistrement d'un tissu local autour de la zone d'intérêt de la première image, qui correspond à une zone de la deuxième image,
- un moyen de génération d'une troisième image ;
dans lequel la troisième image est l'image fluoroscopique augmentée,
dans lequel la troisième image correspond à la deuxième image provenant de la deuxième modalité d'imagerie incluant une zone d'intérêt mise en évidence,
dans lequel la troisième image est obtenue en ajoutant au moins un aspect tridimensionnel d'informations à la deuxième image,
dans lequel ledit au moins un aspect tridimensionnel d'informations correspond aux données anatomiques et/ou aux données de planification extraites de la première image et/ou de l'image virtuelle compatible,
- un dispositif de surveillance configuré pour afficher la troisième image.

2. Système selon la revendication 1,
comprenant en outre un moyen d'exécution d'un enregistrement en plusieurs phases,
dans lequel au moins un objet sensiblement statique est enregistré en premier ; et
dans lequel au moins un objet dynamique est enregistré en deuxième, dans lequel ledit au moins un objet dynamique comprend un diaphragme, une bronche, un vaisseau sanguin ou une combinaison quelconque de ceux-ci, et dans lequel l'objet statique comprend des côtes.

3. Système selon la revendication 1 ou 2,
comprenant en outre un moyen de désaccentuation d'au moins une structure d'interférence sur la troisième image, dans lequel ladite au moins une structure d'interférence est une structure quelconque qui pourrait interférer avec un focus anatomique d'une procédure.

4. Système selon la revendication 1 ou 2, comprenant en outre :
- un tube à rayons X destiné à générer des faisceaux de rayons X ;
- un collimateur qui est conçu pour rétrécir les faisceaux de rayons X générés par le tube à rayons X ;
- un intensificateur d'image à rayons X destiné à absorber des faisceaux de rayons X atténués ; et
- un bras en C pour un déplacement d'un cadre avec une paire fluoroscopique constituée d'un tube à rayons X et d'un intensificateur attachés.

5. Système selon la revendication 4,
comprenant en outre un moyen de conversion d'une image fluoroscopique de données brutes en une image visible, dans lequel l'image fluoroscopique de données brutes est formée par l'intensificateur d'image à rayons X et l'image visible est la deuxième image qui est l'image fluoroscopique peropératoire.

6. Système selon la revendication 4 ou 5,
comprenant en outre une station de planification destinée à planifier la procédure de diagnostic et/ou de traitement sur la base de la première image qui est l'image préopératoire de tomographie assistée par ordinateur, et destinée à générer des données de planification.
